# EUROPEAN PATENT APPLICATION

(11) **EP 4 455 300 A1**
(43) Date of publication of application: **30.10.2024**
(21) Application number: 22909951.0
(22) Date of filing: 19.12.2022
(51) Int. Cl.: C12Q 1/68, C12N 15/10, C12M 1/00

(54) **PREPARATION METHOD FOR MICROSPHERE CHIP AND RELATED APPLICATION**

(30) Priority: 21.12.2021 CN 202111572805; 15.02.2022 CN 202210137482; 25.04.2022 CN 202220960413 U; 19.09.2022 CN 202211139445; 19.09.2022 CN 202211139855; 31.10.2022 CN 202222884503 U
(71) Applicant: Beijing Biomarker Technology Co., Ltd, Beijing 101399 (CN)
(72) Inventor: ZHENG, Hongkun, Beijing 101399 (CN); LIU, Min, Beijing 101399 (CN); ZHANG, Menglong, Beijing 101399 (CN); LI, Jia, Beijing 101399 (CN)
(74) Representative: Barker Brettell LLP
(86) International application number: PCT/CN2022/140097
(87) International publication number: WO 2023/116639

(57) **Abstract**

A method for preparing a microsphere chip and an application thereof. The method for preparing a microsphere chip includes using a silicon dioxide glass slide etched with micropores, as a substrate, uniformly smearing a layer of an ultraviolet adhesive on the silicon dioxide glass slide, then adding coded silicon dioxide microspheres, and centrifugating to obtain a biological chip having a spatial decoding capability.

## Description

### Technical Field

The present invention relates to the field of biological chip processing, and specifically to a method for preparing a microsphere chip and a related application.

### Background

Biological chip technology originates from molecular hybridization of nucleic acids. Biological chips generally refer to micro-arrays of biological information molecules (such as gene fragments, DNA fragments or peptides, and proteins) immobilized at high density on mutually supportive media. The sequence and position of each molecule in the array are known and are pre-determined sequential dot matrixes. Biological chip technology is one of the most promising technologies for DNA analysis, which may be applied to nucleic acids, proteins, cells, tissue and so on. At present, disease diagnosis with the biological chips is still in the research stage all over the world. However, biological chips have been used abroad to observe expression and mutation of oncogenes and some genetic disease genes such as muscular atrophy.

Randomly adding coded microspheres to microstructurally-processed chips is a method of processing the biological chips, but the method results in an upper limit on the drop hole efficiency of the microspheres due to errors in chip-etched apertures and uneven sizes of the microspheres. Generally, the microsphere drop hole rate is not high, and the chip has more residual microspheres.

In recent years, a spatial gene expression technology develops rapidly, and one of the most mature commercial platforms is Visium from 10×Genomics, which enables gene expression profiling in a morphological context. However, the high cost of the Visium hinders the promotion of its application.

### Summary

The present invention is mainly intended to provide a method for preparing a microsphere chip and its related applications, so as to solve problems of complex processes, low drop hole rates, and poor stability of biological chip processing technologies in the prior art.

In order to achieve the objectives of the present invention, the present invention provides a method for preparing a method for preparing a microsphere chip (specifically a silicon dioxide chip using a microsphere self-assembly technique). The method includes the following steps:
1) cleaning a silicon dioxide glass slide with micropores;
2) uniformly spin-coating an ultraviolet curing adhesive on a surface of the cleaned glass slide, then performing ultraviolet radiation, and forming a uniform thin film on the surface of the glass slide;
3) preparing a silicon dioxide microsphere solution with a concentration of 2×10⁵-3×10⁵/µL, then dropwise adding the microsphere solution in the middle of the glass slide with the thin film, and opening a spin-coating apparatus for centrifugation, so as to make microspheres drop into micropores of the glass slide;
4) removing liquid left on the surface of the glass slide, washing with ultrapure water, and then drying; and
5) cleaning the surface of the glass slide with a brush.

In the present invention, the diameters of the micropores are similar to the diameters of the silicon dioxide microspheres. The size of the microspheres should be ensured to match the etched micro-holes on the chip.

The Main components of the ultraviolet curing adhesive include base resin, an active monomer, and a photoinitiator, etc., for example, a Feifanli 3217 UV glue produced by Mizhan Technology Co., Ltd., and may also be purchased from Taobao manufacturers such as KAFUTER and RONGTAI, preferably Feifanli.

The thickness of the thin film is approximately 1-3 µm (preferably 1 µm, thicknesses below 1 µm are not excluded here, such as 1 nm, 10 nm, 15 nm, 20 nm, 30 nm, 50 nm, 60 nm, 70 nm, 80 nm, or 90 nm, etc.) The thickness of the ultraviolet curing adhesive applied by spin coating should be as thin as possible and evenly spread.

The depths of micropores on the silicon dioxide glass slide are 0.5-2.5 µm, preferably 1.5-2.5 µm (preferably about 2.1 µm, more preferably 1.5 µm, thicknesses below 0.5 µm are not excluded here, so as to match smaller sizes of the microspheres). The micropores are uniformly distributed on the glass slide, with a distance between centers of the two adjacent micropores being 4-6 µm (preferably about 5 µm, distances between central points below 4 µm are not excluded here, such as 2.5 um, 3 um, 3.5 um, etc.), calculated according to a total area of 7 mm×7 mm of the glass slide.

In the foregoing method, centrifugation in step 3) is performed with a plate centrifuge at a rotary speed of 1000 rpm-3000 rpm (preferably 2000 rpm) for 10 seconds to 1 minute (preferably 30s).

In the foregoing method, a reagent for preparing the silicon dioxide microsphere solution in step 3) is the ultrapure water, a DMSO solution with a volume percent concentration of 5-20%, or an ultraviolet curing adhesive solution with a volume percent concentration of 2.5-10‰. Preferably, the ultraviolet curing adhesive solution has a concentration of 2.5-10‰, and more preferably, a concentration of 5‰.

In the foregoing method, step 1) includes soaking the silicon dioxide glass slide in a Piranha Solution for 30 minutes, then cleaning successively with the ultrapure water and anhydrous ethanol, and subsequently air drying.

The Piranha Solution is a mixture of a concentrated sulfuric acid and a hydrogen peroxide solution in a volume ratio of 7:3.

In the present invention, the silicon dioxide microspheres are covalently bonded with nucleic acids, proteins, or peptides.

By means of the above technical solutions, the present invention has at least the following advantages and beneficial effects.
(I) In the present invention, using the ultraviolet curing adhesive (UV adhesive) to process a silicon dioxide chip for assembling microspheres can achieve a drop hole rate of more than 99% with fewer impurities.
(II) The Instrument consumables used are all conventional procurement supplies, and compared with mature foreign imported chips, production costs are greatly reduced.
(III) Rapid assembly is achieved, and the entire microsphere chip assembly flow is controlled within 1 hour, such that the method is suitable for factory production and manufacturing.
(IV) The microsphere chip provided in the present invention is particularly suitable for use as a spatial transcriptome chip.

In some preferred embodiments of the present invention, the silicon dioxide microspheres are silicon dioxide microspheres covalently bonded with long coding sequences. Further provided is a method for preparing the silicon dioxide microspheres with long coding sequences. The method includes the following steps.
(1) Four groups of primers are designed, which respectively are a primer 1, a primer 2, a primer 3, and a primer 4. The primers are all single-stranded oligonucleotides. Annealing can be performed between the primer 2 and the primer 3, and the lengths of sequences of the primer 1 and the primer 4 are the same.
   Primer 1 has an amino group modification at 5' end, and comprises a READ1 sequence, a barcode 1 sequence, and a linker 1 sequence of an Illumina sequencing platform from 5' to 3'; the barcode 1 sequence is a coding sequence (including 12-768 sequences) with a length of 10-20 nt, and the linker 1 sequence is an auxiliary connection sequence with a length of 1-20 nt.
   Primer 2 is a coding sequence with a length of 10-20 nt, i.e. a barcode 2 sequence (including 12-768 sequences).
   Primer 3 is 12-60 nt long and comprises, a reverse complementary sequence of a linker 2 sequence, a reverse complementary sequence of the barcode 2 sequence, and a reverse complementary sequence of the linker 1 sequence from 5' to 3'.
   Primer 4 comprises the linker 2 sequence, a barcode 3 sequence (including 12-768 sequences), a UMI sequence (random sequence), and a Poly T sequence from 5' to 3'; and the barcode 3 sequence is a coding sequence with a length of 10-20 nt, the UMI sequence is a random primer with a length of 8-16 nt (used for distinguishing different transcripts), and the Poly T sequence has a length of 10-35 nt and comprises a VN sequence at the end, wherein V and N are degenerate bases, V represents A, G or C, and N represents A, T, G, or C;
(2) activation of carboxylated silicon dioxide microspheres: placing the carboxylated silicon dioxide microspheres in an EDC and NHS mixed solution for activation;
(3) mixing the activated microspheres and the primer 1, and performing a condensation reaction to obtain microspheres with different sequences;
(4) performing an annealing reaction between the primer 2 and the primer 3;
(5) mixing an annealing product obtained in step (4) and the microspheres obtained in step (3), and performing a DNA strand extension reaction; and
(6) mixing the microspheres obtained in step (5) and the primer 4, performing a ligation reaction, thereby obtaining the silicon dioxide microspheres with long coding sequences.

In the present invention, the primer 1 serves as a primer for the first round, the annealing product formed by the primers 2 and 3 serves as a primer for the second round, and the primer 4 serves as a primer for the third round.

When designing primers, not only should the principle of complementary base pairing be considered, but it is also important to avoid the formation of stable dimers or hairpin structures within or between primers.

Further, step (2) includes: centrifugating 4-10 mL of the carboxylated silicon dioxide microspheres with a concentration of 0.1-0.5 mg/mL, then precipitating and mixing with 4-10 mL of the EDC and NHS mixed solution, and oscillating overnight at room temperature and 300-2000rpm.

Here, a method for preparing the EDC and NHS mixed solution comprises: dissolving 10-30 mg of EDC and 5-30 mg of NHS in 1000-10000 µL of 0.1-1M MES, so as to obtain the EDC and NHS mixed solution.

Further, step (3) comprises: adding the activated microspheres to a 96-well plate, with 10-40 µL per well, additionally adding 2-10 µL of a primer 1 solution to each well, and oscillating overnight at 20-30°C and 300-2000 rpm; after the reaction ends, washing the microspheres with PBS containing 0.001-0.03% v/v tween-20, then washing the microspheres with a TE buffer, and resuspending the cleaned microspheres with enzyme-free water.

The primer 1 solution is prepared by dissolving the primer 1 in 0.1-1 M MES buffer, resulting in a final concentration of 10-100 µM for the Primer 1 solution.

Further, step (4) includes: mixing the primer 2 and the primer 3 in an equal molar ratio, adding a 5× annealing buffer for an annealing reaction; a condition for the annealing reaction comprises: 95°C -15°C, annealing by 1-10°C every 1-3 minutes, so as to obtain an annealing product.

The 5× annealing buffer comprises: 10-50 µL of a 1M Tris-HCl solution, 5-20 µL of 0.5M EDTA, and 50-150 µL of 2M NaCl, making up to 1000 µL with double distilled water.

Further, step (5) includes: adding the microspheres obtained in step (3) to a 96-well plate, with 10-40 µL per well, and additionally adding, to each well, 5 µL of a 5×T4 ligation buffer, 2-10 µL of 100-1000U/µL T4 ligase, and 2-10 µL of 20-100µM the annealing product, making up to 50 µL with double distilled water; oscillating at 16°C and 300-2000rpm for 0.5-3 hours; and then washing the microspheres with a 5-20mM pH8 Tris-HCl solution, and resuspending the cleaned microspheres with enzyme-free water.

Further, step (6) includes: adding the microspheres obtained in step (5) to a 96-well plate, with 10-40 µL per well, and additionally adding, to each well, 5 µL of a 5×T4 ligation buffer, 2-10 µL of 100-1000U/µL T4 ligase, and 2-10 µL of a primer 4 solution, making up to 50 µL with double distilled water; oscillating at 16°C and 300-2000rpm for 0.5-3 hours; and then washing the microspheres with a 10mM pH 8 Tris-HCl solution, placing the cleaned microspheres in a 0.1-2M NaOH solution for DNA denaturation, then washing the microspheres with enzyme-free water, finally resuspending the cleaned microspheres with a TE-TW solution, and performing preservation at 4°C.

The primer 4 solution is prepared by dissolving the primer 4 in a TE buffer with pH 8.0, resulting in a final concentration of 10-100 µM for the primer 4 solution.

The TE-TW solution is a TE buffer containing 0.01% Tween-20.

The T4 ligase used in the present invention may be purchased from companies such as Vazyme, Sangon, Yeasen, NEB, etc.

The silicon dioxide microspheres prepared in the preferred embodiments of the present invention may be used for further DNA decoding and applied to spatial transcriptome sequencing.

The method for preparing the silicon dioxide microspheres with long coding sequences has at least the following advantages and beneficial effects.
(I) A variety of microspheres are prepared by this method, utilizing 12-768 types of barcode 1, barcode 2, and barcode 3, ultimately yielding microspheres carrying 1,728 (12×12×12) or even 452,984,831 (768×768×768) different sequences. This enables more comprehensive tissue localization in spatial transcriptome sequencing, revealing transcriptomic differences between various regions within the tissue.
(II) The method for preparing the silicon dioxide microspheres coded with long sequences provided in the preferred embodiments is simple and easy to operate, and compared with a traditional microsphere synthesis method, a loss rate of the microspheres is reduced.
(III) The method provided in the preferred embodiments is high in uniformity, the barcode 1, the barcode 2, and the barcode 3 of the present invention are relatively uniform, and the concentrations of the primers are strictly quantified, such that random increase or decrease of a specific barcode is effectively prevented.
(IV) The method provided in the preferred embodiments improves the microsphere synthesis reaction system, ensuring that the microspheres and the primers are in full contact with each other and ligated through the T4 ligase, such that ligation efficiency at each step is relatively high, ensuring the complete extension of the DNA sequence.
(V) In the preferred embodiments, silicon dioxide microspheres are used as marker carriers. The nano silicon dioxide microspheres are a non-toxic, non-polluting, and high-strength inorganic non-metallic nanomaterial with good stability and a large surface area. Nanoscale monodisperse silicon dioxide microspheres may be prepared on a certain scale, and have already been widely applied in biological cell separation and medical engineering. Because of the characteristics of being good in shape uniformity, controllable in size, good in dispersibility, single in composition, and easy in surface functionalization, the monodisperse silicon dioxide microspheres have significant application value in synthesis of the silicon dioxide microspheres coded with long sequences.

In some preferred embodiments, the silicon dioxide microspheres are silicon dioxide microspheres covalently bonded with long DNA sequences. Further provided is a method for preparing the silicon dioxide microspheres with long DNA sequences. The method includes the following steps:
(1) designing four groups of primers, which respectively are a primer 1, a primer 2, a primer 3 and a UMI primer, wherein the primers are all single-stranded oligonucleotides, the lengths of the primer 1, the primer 2, and the primer 3 are the same, with a GC content between 45% and 55%, ensuring that Tm values of each primer are similar;
   the primer 1 (a primer for the first round) has an amino group modification at 5' end, and comprise a READ1 sequence of an Illumina sequencing platform, a barcode 1 sequence and a linker 1 sequence from 5' to 3' (the whole sequence are synthesize by the other three rounds of primers in a way of reverse complementary annealing); the primer 2 (a primer for the second round) comprises a reverse complementary sequence of a linker 2 sequence, a reverse complementary sequence of a barcode 2 sequence, and a reverse complementary sequence of the linker 1 sequence from 5' to 3'; the primer 3 comprises a reverse complementary sequence of a linker 3 sequence, a reverse complementary sequence of a barcode 3 sequence, and a reverse complementary sequence of the linker 2 sequence from 5' to 3'; the UMI primer comprises a polyA sequence, a UMI sequence (random sequence), and a reverse complementary sequence of the linker 3 sequence from 5' to 3'; and wherein the barcode 1 sequence, the barcode 2 sequence, and the barcode 3 sequence are different barcode sequences, and a length of the poly A sequence is 20-35 nt;
   When designing primers, on one hand, the principle of reverse complementary synthesis mentioned above must be considered. On the other hand, it is required that the various barcode sequences have significant differences from each other and that there are no identical sequences on the genome.
(2) Activation of carboxylated silicon dioxide microspheres: placing the carboxylated silicon dioxide microspheres in an EDC and NHS mixed solution for activation;
(3) Ligation reaction: putting the activated microspheres in a primer 1 solution, and performing a condensation reaction to obtain silicon dioxide microspheres with different sequences;
   wherein, the amino-modified oligonucleotide chain solution contains the READ1 sequence, the barcode 1 sequence, and the linker 1 sequence;
(4) Synthesis of the long DNA sequences: mixing the microspheres obtained in step (3), the primer 2, and a polymerization reaction reagent, performing a DNA strand extension reaction, and then removing the reverse complementary sequence of the linker 1 sequence; mixing the obtained microspheres, the primer 3, and the polymerization reaction reagent, further performing the DNA strand extension reaction, and then removing the reverse complementary sequence of the linker 2 sequence; and mixing the microspheres, the UMI sequence, and the polymerization reaction reagent, further performing the DNA strand extension reaction, and then removing the reverse complementary sequence of the linker 3 sequence, so as to obtain the silicon dioxide microspheres with long DNA sequences.

Step (2) includes: centrifuging 50 mg/mL of the carboxylated silicon dioxide microspheres, then precipitating and mixing the carboxylated silicon dioxide microspheres and 20-100 µL of an EDC and NHS mixed solution, and oscillating for reaction at room temperature at 1500-2000rpm for 30 min-1 hour.

Wherein, a method for preparing the EDC and NHS mixed solution includes: dissolving 1.09 mg of EDC and 0.65 mg of NHS in 100 µL of 0.1M MES to obtain the EDC and NHS mixed solution.

Step (3) includes: uniformly mixing the activated microspheres and 2.5 µL of an oligonucleotide chain solution modified by 50µM amino, and oscillating for reaction overnight at room temperature at 2000rpm; After the reaction is complete, centrifuging, collecting the microspheres, and washing the microspheres for subsequent synthesis reaction.

Washing the microspheres comprises: placing the microspheres in 0.1M PBS containing 0.02% Tween 20, centrifugating and collecting the microspheres, and then washing the microspheres with a TE buffer with pH 8.0 for twice.

The polymerization reaction reagent in step (4) contains dNTPs, Klenow enzyme, and a Klenow enzyme reaction buffer.

A reaction system for performing DNA strand extension includes 1 µL of the 50µM primer 2, 5 µL of the 10×Klenow enzyme reaction buffer, 4 µL of the 2.5mM dNTPs, and 1 µL of the 5U/µL Klenow enzyme.

Reaction conditions includes oscillating for reaction at 37°C at 2000rpm for 30 min-1 hour.

A reaction system for the strand extension of the UMI primer and the microspheres in step (4) includes 1 µL of the 50µM UMI primer, 5 µL of the 10×Klenow enzyme reaction buffer, 4 µL of the 2.5mM dNTPs, and 1 µL of the 5U/µL Klenow enzyme.

Reaction conditions includes oscillating for reaction at 37°C at 2000rpm for 0.5-1 hour.

Preferably, a nucleotide sequence of the primer 1 is shown in SEQ ID NOs: 1-5, a nucleotide sequence of the primer 2 is shown in SEQ ID NOs: 6-10, a nucleotide sequence of the primer 3 is shown in SEQ ID NOs: 11-15, and a nucleotide sequence of the UMI primer is shown in SEQ ID NO: 16.

The carboxylated silicon dioxide microspheres used in the silicon dioxide microspheres with long DNA sequences are purchased from Shanghai So-Fe Biomedicine Technology Co., Ltd., and may also be prepared according to conventional methods.

The solution for preparing the silicon dioxide microspheres with long DNA sequences has at least the following advantages and beneficial effects:
(I) This preferred embodiment provides a method for synthesizing a carboxylated silicon dioxide coded with a long sequence. The method uses silicon dioxide as a carrier and employs a chemical reagent to activate a surface carboxyl functional group, which may be covalently bonded with an amino-modified oligonucleotide sequence. Furthermore, subsequent sequences (for sequence extension) may be introduced in a way of complementary sequence annealing combined with a polymerization reaction. Various silicon dioxide microspheres coded with long sequences can be obtained with the method. Compared with traditional methods, the advantages of the method lie in that, the silicon dioxide microspheres are modified with more oligonucleotide sequences and contain more comprehensive biological information.
(II) High activation efficiency: EDC and NHS can fully activate carboxyl structures modified on the surfaces of the silicon dioxide microspheres.
(III) High ligation efficiency: an amino oligonucleotide chain can be effectively connected to the carboxyl microspheres through the condensation reaction.
(IV) High operation success rate: the operation process is simple, using minimal reagents to ensure the maximum synthesis efficiency.

In a preferred embodiment of the present invention, a spatial transcriptome chip is further provided to address the deficiency of lower resolution and higher cost in sample analysis of biological chips in the prior art. This is achieved by improving the resolution of sample analysis through the use of microporous structures.

The spatial transcriptome chip provided in the present invention includes:
a substrate, a plurality of rectangular microporous regions are formed on the substrate, a plurality of sub-rectangular microporous regions are formed within the rectangular microporous regions, a plurality of microporous structures are uniformly distributed in each sub-rectangular microporous region, the microporous structure is configured to place coded microspheres.

According to the spatial transcriptome chip provided in the present invention, a gap is formed between the adjacent sub-rectangular microporous regions, and the size of each gap is the same.

According to the spatial transcriptome chip provided in the present invention, the width of the gap ranges from 2 µm to 20 µm.

According to the spatial transcriptome chip provided in the present invention, at least one of the sub-rectangular microporous regions located at each of the four right angles of the rectangular microporous region is provided with a marker.

According to the spatial transcriptome chip provided in the present invention, notches are formed in the sub-rectangular microporous regions located in any three of the four right angles of the rectangular microporous region, so as to form the marker.

According to the spatial transcriptome chip provided in the present invention, the notch is located at any one of the right angles of the sub-rectangular microporous region.

According to the spatial transcriptome chip provided in the present invention, the diameter of the microporous structure ranges from 1 µm to 10 µm.

According to the spatial transcriptome chip provided in the present invention, the length and width of the rectangular microporous region both range from 5 mm to 20 mm.

According to the spatial transcriptome chip provided in the present invention, the length and width of the sub-rectangular microporous region all range from 100 µm to 300 µm.

According to the spatial transcriptome chip provided in the present invention, the substrate is a glass substrate, and the glass substrate is etched to form the microporous structure.

According to the spatial transcriptome chip provided in the present invention, the plurality of rectangular microporous regions are constructed on the substrate, the plurality of sub-rectangular microporous regions are also formed within each rectangular microporous region, and the microporous structures are uniformly distributed in each sub-rectangular microporous region. The resolution of a spatial transcriptome is improved through the microporous structures to realize high sensitivity detection and meet the requirements of scientists for subcellular structure analysis. Moreover, glass substrates can be used, and the microporous structures may be manufactured using an etching technology, which t simplifies the manufacturing process and reduces consumable costs.

In some other preferred embodiments of the present invention, a spatial transcriptome biological chip is further provided to solve the deficiency that bright-field microscopic imaging such as HE staining, toluidine blue staining and Masson staining, which cannot be performed on non-transparent substrates. This chip also addresses the limitation of glass slide substrates in meeting the requirements of scientists for subcellular structure analysis.

The spatial transcriptome biological chip provided in some preferred embodiments includes:
a transparent substrate, the transparent substrate is subjected to photolithography and etching to form a microporous region, a microporous structure is formed within the microporous region, so as to place coded microspheres with primers; at least one sub-region is formed in the microporous region, the sub-region is a circular region or a polygonal region.

According to the spatial transcriptome biological chip provided in the present invention, a plurality of micro-units are formed within the sub-region, the adjacent micro-units are arranged at intervals, and the micro-units are repeatedly arranged in circles or polygons.

According to the spatial transcriptome biological chip provided in the present invention, a notch is formed at an edge and/or a corner of the micro-unit, so as to form an azimuth marker.

According to the spatial transcriptome biological chip provided in the present invention, a distance between the micro-units is between 0 µm and 40 µm.

According to the spatial transcriptome biological chip provided in the present invention, a diameter of the microporous structure ranges from 0.1 µm to 10 µm, and a distance between central points of adjacent two micropores is between 0.1 µm and 20 µm.

According to the spatial transcriptome biological chip provided in the present invention, a long side of the transparent substrate ranges from 10 to 100 mm, and a short side ranges from 5 to 50 mm.

According to the spatial transcriptome biological chip provided in the present invention, a size of the sub-region ranges from 9mm² to 1875mm².

According to the spatial transcriptome biological chip provided in the present invention, the transparent substrate is one of glass, quartz, plastic, magnesium chloride and gallium arsenide.

According to the spatial transcriptome biological chip provided in the present invention, the microporous structure includes an expansion portion located on a surface of the transparent substrate and a shrinkage portion located inside the transparent substrate, the expansion portion and the shrinkage portion are interconnected along a depth direction of the transparent substrate, thereby forming the microporous structure on the transparent substrate.

According to the spatial transcriptome biological chip provided in the present invention, the transparent substrate comprises a first surface and a second surface, both of which are respectively subjected to photolithography and etching to form the microporous regions. Additionally, the first surface and the second surface are two opposite surfaces of the transparent substrate.

The spatial transcriptome biological chip provided in the preferred embodiments of the present invention uses the transparent substrate, the microporous structures are manufactured with the photolithography and etching technology, and the microporous regions are formed, such that the biological chip is simple in manufacturing process, and consumable costs are reduced. Furthermore, through bright-field imaging, an analysis effect of the spatial transcriptome is greatly improved by combining with a gene expression result. In addition, the microporous structures of the chip are high in resolution, such that subcellular-level spatial transcriptome data analysis can be realized.

In some preferred embodiments of the present invention, a three-color fluorescence decoding method based on a subcellular-level spatial chip is further provided. The method includes the following steps:
A, preparation of a biological chip: immobilizing silicon dioxide microspheres, connected with a primer 1, a primer 2, and a primer 3, at well positions of a well plate, so as to obtain the biological chip;
B, hybridization of the chip and a decoding probe: mixing a decoding probe I, a decoding probe II, and a decoding probe III, which have different fluorescent tags, then performing a hybridization reaction with the biological chip, and then detecting corresponding fluorescence signals;
C, DNA denaturation and cleaning: denaturing the hybridized biological chip with sodium hydroxide, washing and drying the melted biological chip, and then re-hybridizing a next round of decoding probes again; and
D, repeating steps B-C for 2-9 times, and achieving chip decoding;

Wherein the primer 1, the primer 2, and the primer 3 comprise 4-384 primer sequences respectively; the primer 1, the primer 2, and the primer 3 are connected in series in sequence according to 5'-3'; and
the decoding probe I is a single-stranded oligonucleotide complementary to a barcode of the primer 1, the decoding probe II is a single-stranded oligonucleotide complementary to a barcode of the primer 2, and the decoding probe III is a single-stranded oligonucleotide complementary to a barcode of the primer 3.

Preferably, the silicon dioxide microsphere in step A are carboxylated silicon dioxide microspheres.

Further, step A includes: connecting each of the 4-384 primer sequences of the primer 1 to the carboxylated silicon dioxide microspheres through an amino-carboxyl condensation reaction, mixing and cleaning the microspheres after connection is completed, and uniformly dividing the microspheres into 4-384 portions; performing a connection reaction on each portion added with 4-384 primer sequences of the primer 2, mixing and cleaning the microspheres after connection is completed, and uniformly dividing the microspheres into 4-384 portions; performing the connection reaction on each portion added with 4-384 primer sequences of the primer 3, uniformly mixing and spreading the microspheres on a microporous glass plate after connection is completed, and immobilizing the microspheres at the well positions, so as to obtain the biological chip.

The fluorescent tag used in the present invention may be selected from DAPI, FITC, Alexa fluor 488, Cy2, Cy3, Cy5, Cy5.5, TRITC or Cy7, etc.

Further, the hybridization reaction in step B is performed in a hybridization buffer; and components of the hybridization buffer comprise 1-10mM NaCl, 2-5mM Tris-HCl, 1-3mM MgCl₂, and 0.5-5mM Dithiothreitol (DTT).

In step B, a concentration of the mixing decoding probe is 1nM-50nM, 10 µL of the mixed decoding probe is mixed with 40-190 µL of the hybridization buffer, and the mixture is added to the well positions, so as to perform the hybridization reaction with the biological chip.

A condition of performing the hybridization reaction in step B comprises: performing the reaction at 37-60°C for 5-20 min.

Further, step C includes the following steps:
C1, 100 µL of a 0.1-2M sodium hydroxide solution is added to each well position, the solution is allowed to stand for 1-10 minutes, and then the solution is poured in the well positions;
C2, step C1 is repeated for 2-3 times to ensure complete DNA denaturation; and
C3, the melted biological chip is cleaned with enzyme-free water for 1-3 times, drying is performed, and then a next round of decoding probes is hybridized again.

In addition, an application of the method provided in the preferred embodiments in spatial omics research at a subcellular level (especially research on tissue spatial position information at a subcellular level) is further provided.

The preferred embodiments have at least the following advantages and beneficial effects.
(I) Fast decoding speed: the hybridization time between the probe and the chip is shorter, and the fluorescence signal is collected quickly, with a faster round of fluorescence collection.
(II) Containing more sequences: the microspheres are available in a variety of combinations and thus contain a large number of sequences that may be used for decoding.
(III) High operation success rate: an operation process is simple, and fewer reagents are used, which ensures the highest possible synthesis efficiency.

### Brief Description of the Drawings

The drawings, which form a part of the present application, are used to provide a further understanding of the present invention. The exemplary embodiments of the present invention and the description thereof are used to explain the present invention, but do not constitute improper limitations to the present invention. In the drawings:
Fig. 1 is a schematic flowchart of a method for assembling a silicon dioxide chip and microspheres according to the present invention.
Fig. 2 is an effect diagram of a silicon dioxide chip before assembly according to a preferred embodiment of the present invention.
Fig. 3 is an effect diagram of a silicon dioxide chip and microspheres after self-assembly according to a preferred embodiment of the present invention.
Fig. 4 is an effect diagram of a silicon dioxide chip processed for 30 min at 95°C according to a preferred embodiment of the present invention.
Fig. 5 is a schematic diagram of a sequence structure of silicon dioxide microspheres coded with long sequences synthesized by a connection method according to the present invention.
Fig. 6 is a fluorescence quality testing diagram of silicon dioxide microspheres coded with long sequences synthesized by a connection method according to the present invention.
Fig. 7 is a fluorescence quality testing diagram of silicon dioxide microspheres coded with long sequences synthesized in CN114410764A.
Fig. 8 is a schematic structural diagram of silicon dioxide microspheres coded with long sequences synthesized in Embodiment 7 of the present invention.
Fig. 9 is a schematic structural diagram of a spatial transcriptome chip according to a preferred embodiment of the present invention.
Fig. 10 is a schematic structural diagram of a rectangular microporous region in a spatial transcriptome chip according to a preferred embodiment of the present invention.
Fig. 11 is a schematic structural diagram of a sub-rectangular microporous region in a spatial transcriptome chip according to a preferred embodiment of the present invention.
Fig. 12 is a schematic structural diagram of a first notch formed in a sub-rectangular microporous region according to a preferred embodiment of the present invention.
Fig. 13 is a schematic structural diagram of a second notch formed in a sub-rectangular microporous region according to a preferred embodiment of the present invention.
Fig. 14 is a schematic structural diagram of a third notch formed in a sub-rectangular microporous region according to a preferred embodiment of the present invention.

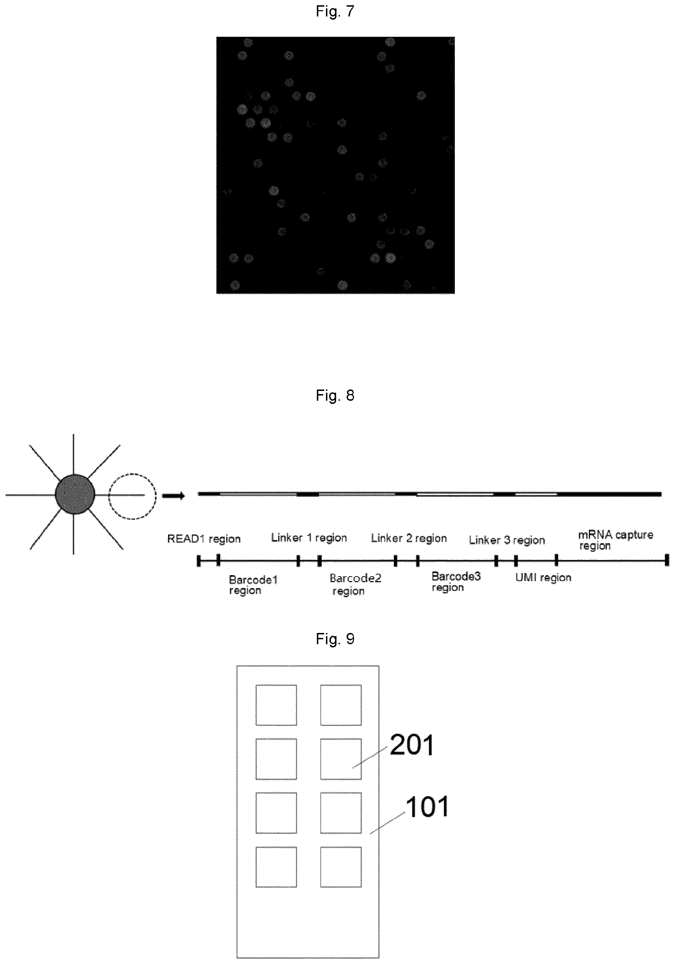

Reference signs in Figs 9 to 14 are as follows:
101: Substrate; 201: Rectangular microporous region; 301: Sub-rectangular microporous region; 70: Microporous structure; 50: Notch; 60: Gap.

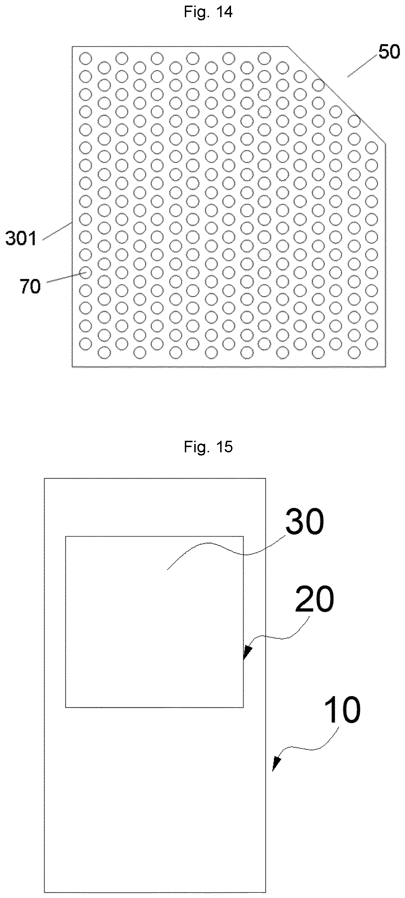

Fig. 15 is a schematic structural diagram of a spatial transcriptome biological chip in which a sub-region is in a large rectangular shape according to a preferred embodiment of the present invention.

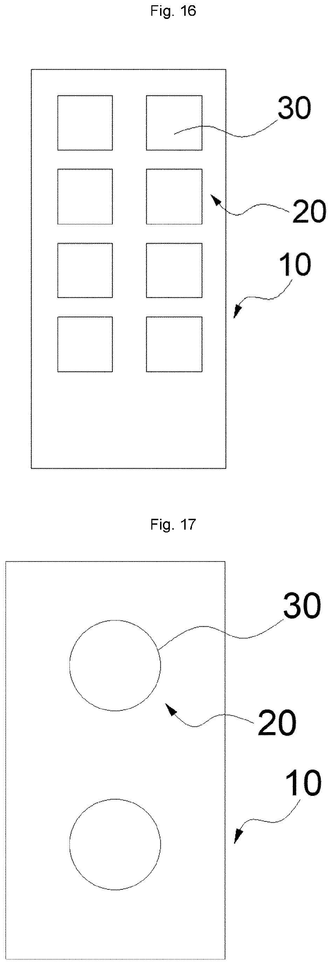

Fig. 16 is a schematic structural diagram of a spatial transcriptome biological chip in which a sub-region is in a small rectangular shape according to a preferred embodiment of the present invention.

Fig. 17 is a schematic structural diagram of a spatial transcriptome biological chip in which a sub-region is in a circle according to a preferred embodiment of the present invention.

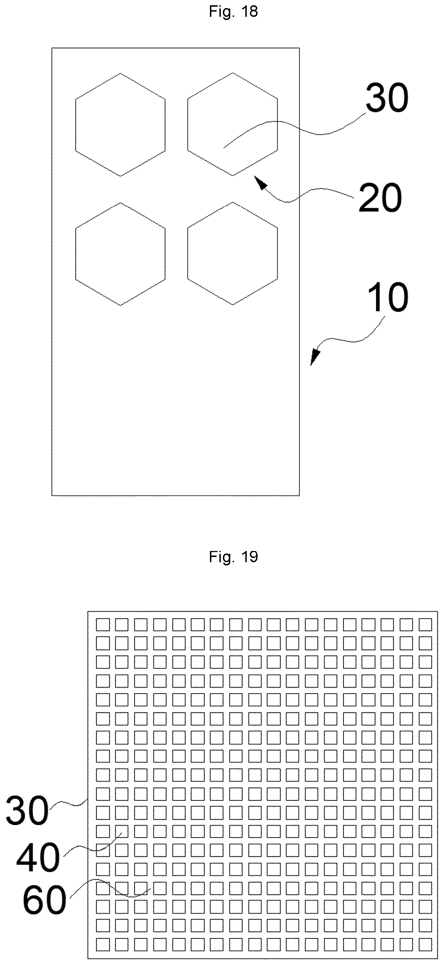

Fig. 18 is a schematic structural diagram of a spatial transcriptome biological chip in which a sub-region is in a hexagon shape according to a preferred embodiment of the present invention.

Fig. 19 is a schematic structural diagram of a spatial transcriptome biological chip in which micro-units are uniformly arranged in a rectangular shape according to a preferred embodiment of the present invention.

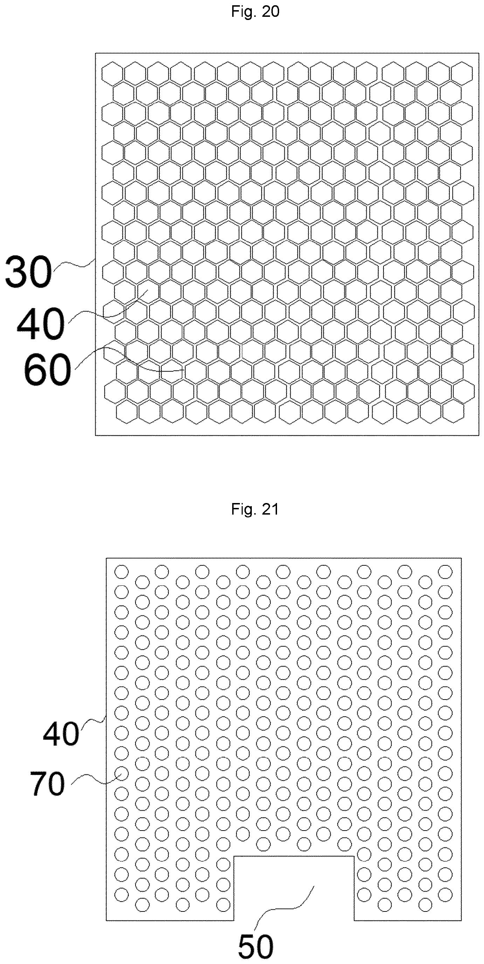

Fig. 20 is a schematic structural diagram of a spatial transcriptome biological chip in which micro-units are uniformly arranged in a hexagon shape according to a preferred embodiment of the present invention.

Fig. 21 is a schematic structural diagram of a spatial transcriptome biological chip in which a notch is located at an edge position of a micro-unit according to a preferred embodiment of the present invention.

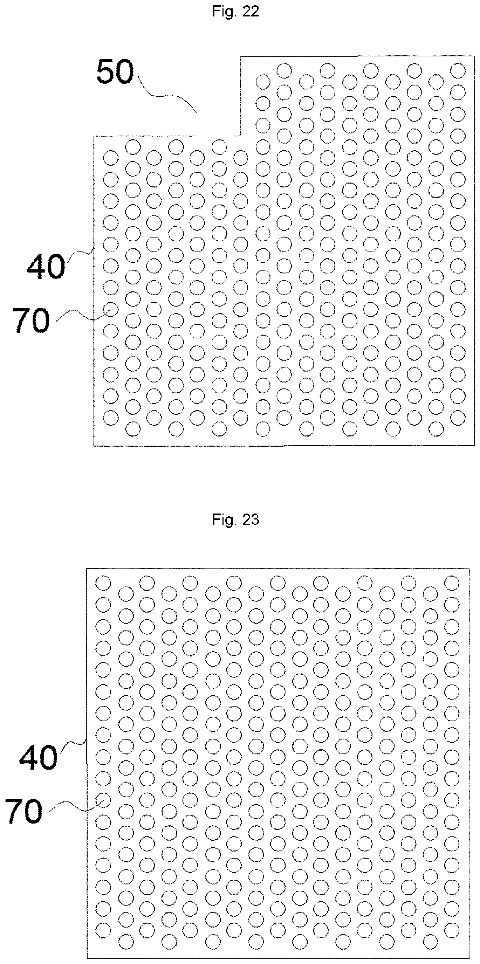

Fig. 22 is a schematic structural diagram of a spatial transcriptome biological chip in which a notch is located at a corner position of a micro-unit according to a preferred embodiment of the present invention.

Fig. 23 is a schematic structural diagram of a spatial transcriptome biological chip that does not form a notch in a micro-unit according to a preferred embodiment of the present invention.

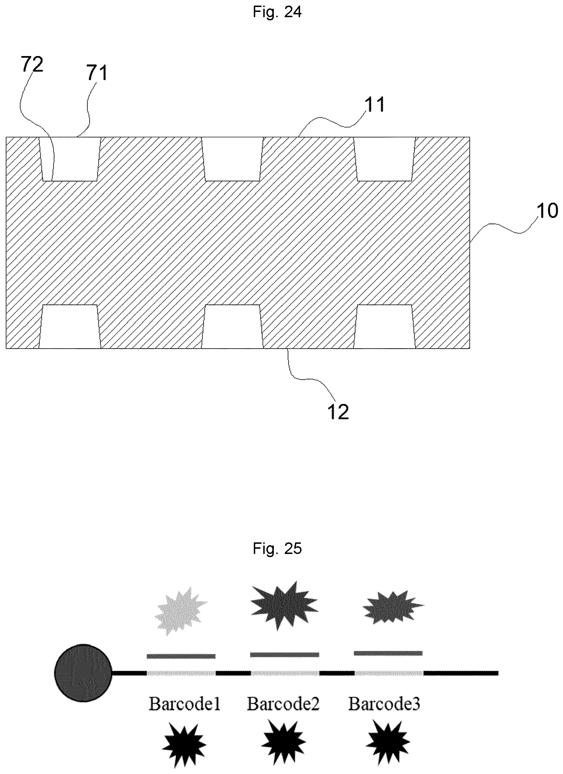

Fig. 24 is a longitudinal cross-sectional view of a spatial transcriptome chip according to a preferred embodiment of the present invention.

Reference signs in Figs 15 to 24 are as follows:
10: Transparent substrate; 11: First surface; 12: Second surface; 20: Microporous region; 30: Sub-region; 40: Micro-unit; 50: Notch; 60: gap; 70: Microporous structure; 71: Expansion portion; 72: Shrinkage portion.

Fig. 25 shows a process of hybridizing microspheres and different decoding probes, then performing arrangement according to colors of each round of fluorescent tags to obtain corresponding sequence structures, so as to achieve chip decoding.

### Detailed Description of the Embodiments

It is to be noted that the embodiments in the present application and the features in the embodiments may be combined with one another without conflict. The present invention will be described below in detail with reference to the embodiments.

A first typical embodiment of the present invention provides a method for assembling a silicon dioxide chip and microspheres. A silicon dioxide glass slide etched with micropores is used as a substrate, a layer of an ultraviolet adhesive (ultraviolet curing adhesive) is uniformly smeared on the substrate, then coded silicon dioxide microspheres are added (i.e., the silicon dioxide microspheres are covalently connected with nucleic acid molecules), and a biological chip having a spatial decoding capability is obtained after centrifugation. The present invention provides a method that is simple and low in cost, and rapid manufacturing of a spatial transcriptome chip is realized.

This embodiment uses the following technical solutions.

A silicon dioxide chip is first cleaned, then UV adhesive coating is performed on the surface of the silicon dioxide chip, then coded free silicon dioxide microspheres are added, centrifugation is performed by a spin-coating apparatus to cause the microspheres to drop on the silicon dioxide chip, and a chip for a spatial transcriptome experiment is finally obtained through drying and cleaning. The chip may be scanned through a microscope to evaluate a drop hole effect.

### Specifically,

1. Chip surface cleaning: the chip is soaked in a Piranha Solution (concentrated sulfuric acid:hydrogen peroxide solution = 7:3), then the chip is washed with a large amount of ultrapure water, and drying is performed for later use.
2. Chip coating processing: a UV adhesive is used to smear a chip target region, and ultraviolet radiation is performed for later use.
3. Microsphere filling: the ultrapure water is used to dilute the coded microspheres, then the microspheres are uniformly spread in the chip target region, standing for 4 min, and then centrifugating with a spin-coating apparatus (plate centrifuge).
4. Chip cleaning: residual suspension above the target region is removed, and cleaning is performed using a brush after drying.
5. Chip quality control: an assembled spatial transcriptome chip is placed under a scanning microscope for photographing and scanning.

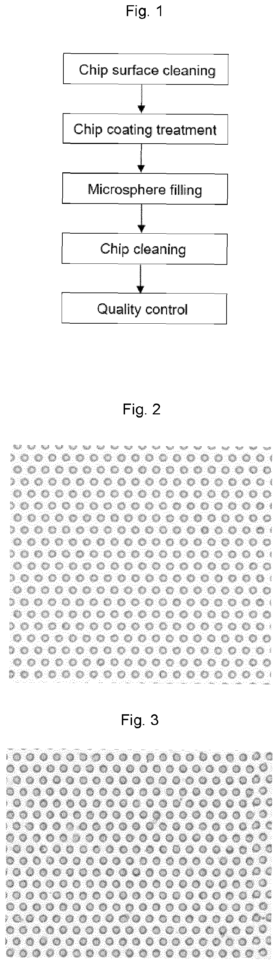

A chip manufacturing flow is shown in Fig. 1.

The following embodiments are used to illustrate the present application, but not to limit the scope of the present invention. If not specifically indicated, the technical means used in the embodiments are conventional means known to those skilled in the art, and raw materials used are commercially available.

The UV adhesive used in the following embodiments is a Feifanli 3217 UV glue produced by Mizhan Technology Co., Ltd. The plate centrifuge is purchased from Tiangen Biochemical Technology (Beijing) Co., Ltd., with a model number being OSE-MP25.

The diameters of the silicon dioxide microsphere are 2.5 µm. The depths of the micropores on the silicon dioxide chip are 2.1 µm. The micropores uniformly distributed on the glass slide, with a distance between centers of the two adjacent micropores is 5 µm, calculated according to a total area 7 mm×7 mm of the glass slide.

Cells and tissue of higher organisms have high spatial heterogeneity. The relative position of a cell in a tissue sample, as well as spatial information on gene expression, is important for studying both disease pathology and biological development. The great increase in resolution and detection throughput of a single-cell sequencing technology enables researchers to obtain heterogeneity between the cells at single-cell resolution. On the contrary, spatial transcriptome, a spatial barcode RNA-Seq method, provides, for the researchers, spatial information of the cells in the tissue and cell compositions and gene expression states of different regions in the tissue. However, the methods in the prior art need to select a marker in advance, and the latest spatial transcriptome technology doesn't yet reach single-cell resolution, such that it is now more common to combine the spatial transcriptome with the single-cell sequencing technology. Generally, a first step of spatial transcriptome sequencing may perform site-directed immobilization on a marker on a biological chip by preparing the marker with a barcode, or performing flows such as marker decoding after random immobilization.

Therefore, in a second preferred embodiment of the present invention, a method for synthesizing silicon dioxide microspheres coded with long sequences by a connection method is further provided. In the method, carboxyl modified silicon dioxide microspheres are used as carriers, and connected with an amino-modified oligonucleotide sequence in a covalent bond manner. Then the binding with a phosphodiester bond between a 5'-P end and a 3'-OH end of the subsequent oligonucleotide sequence is catalyzed by a T4 ligase. By means of the method, various barcode silicon dioxide microspheres can be obtained. Compared with synthesis of Barcode silicon dioxide microspheres by a traditional PCR method, the advantages of the method are that reaction bias is reduced, connection efficiency is improved, and the method is simple and easy to operate, and has wide application prospects.

The present invention provides a new method for synthesizing silicon dioxide microspheres coded with long sequences. First, carboxyl sites on surfaces of the silicon dioxide microspheres are activated to expose surface carboxyl groups, and then a condensation reaction is performed with 12, 24, 48, 96, 192, 288, 384, 768, and even more oligonucleotides with unique Barcode 1 sequences. Phosphodiester bonds are formed between 5'-P ends and 3'-OH ends of the oligonucleotides through catalysis of the T4 ligase, and 12, 24, 48, 96, 192, 288, 384, 768, and even more Barcode 2 sequences are introduced. Finally, the T4 ligase is used again, and 12, 24, 48, 96, 192, 288, 384, 768 Barcode 3 sequences are introduced. After three rounds of reactions, all oligonucleotides on the same microsphere have the same Barcode and different UMI, and the oligonucleotides on different microspheres have different Barcode combinations, such that 1728(12×12×12), even 452984831(768×768×768), silicon dioxide microspheres coded with long sequences are finally obtained.

The present invention uses the following technical solutions.
(1) Primer design: there are 4 groups of primers in total, respectively being a primer 1, a primer 2, a primer 3, and a primer 4. The primers are all single-stranded oligonucleotides. The primer 2 and the primer 3 are complementary sequences, and annealing is performed for later use of double strands. The lengths of sequences of the primer 1 and the primer 4 are the same.
   A first round of the primers: the primer 1, with 5' end being modified by amino, is bonded with carboxyl-modified sites on the surfaces of the silicon dioxide microspheres. From 5'-3', the primer 1 includes a READ1 sequence of an Illumina sequencing platform, a barcode 1 sequence, and a linker 1 sequence. The Barcode 1 sequence is a coding sequence of 10-20 nt; and the linker 1 is an auxiliary connection sequence with a length of 1-20 nt. A second round of the primers includes the primer 2 and the primer 3. The primer 2 is a 10-20 nt single-stranded oligonucleotide for coding, i.e. a Barcode 2 sequence. The primer 3 is a 12-60 nt single-stranded oligonucleotide, which includes a complementary pairing sequence of the primer 2, and an auxiliary connection sequence complementary to the linker 1 and the linker 2. A third round of the primers: the primer 4, from 5'-3' includes a link 2 sequence, a barcode 3 sequence, a UMI sequence (random sequence), and a Poly T sequence. The linker 2 is an auxiliary connection sequence with a length of 1-20 nt. The barcode 3 sequence is a coding sequence of 10-20 nt. UMI is a random primer of 8-16 nt and used for distinguishing different transcripts. Poly T has a length of 10 35 nt and includes a VN sequence (degenerate bases) at the end.
   A complementary pairing principle needs to be taken into consideration during primer design, and a stable dimer or hairpin structure formed between the primers needs to be avoided as well.
(2) Activation of carboxyl-modified silicon dioxide microspheres: proper amount of the carboxyl-modified silicon dioxide microspheres is taken, an EDC/NHS mixed solution is added to activate carboxyl sites on surfaces of the microspheres, and the condensation reaction is performed on subsequent 12, 24, 48, 96, 192, 288, 384, 768, and even more barcode 1 oligonucleotide sequences.
(3) Barcode 1 condensation reaction: in step (2), amino-modified Barcode 1 oligonucleotides (including the READ1 sequence, the Barcode 1 sequence, and the Linker 1 sequence) are added, and a metal bath is oscillated for reaction for 1h, 2h, 4h, 8h, 12h, 24h, etc.
(4) Barcode 2 annealing reaction: the annealing reaction is performed on two complementary strands of the primer 2 and the primer 3 in a PCR instrument.
(5) Barcode 2 and 3 ligation reaction: annealing products in (4) are added to the microspheres in (3) for a DNA strand extension reaction; and the obtained microspheres are added to a Barcode 3 oligonucleotide sequence (including the Linker 2 sequence, the Barcode 3 sequence, the UMI sequence, and the Poly T sequence) for further ligation reaction, and 12×12×12 to 768×768×768 silicon dioxide microspheres coded with long sequences are finally obtained.

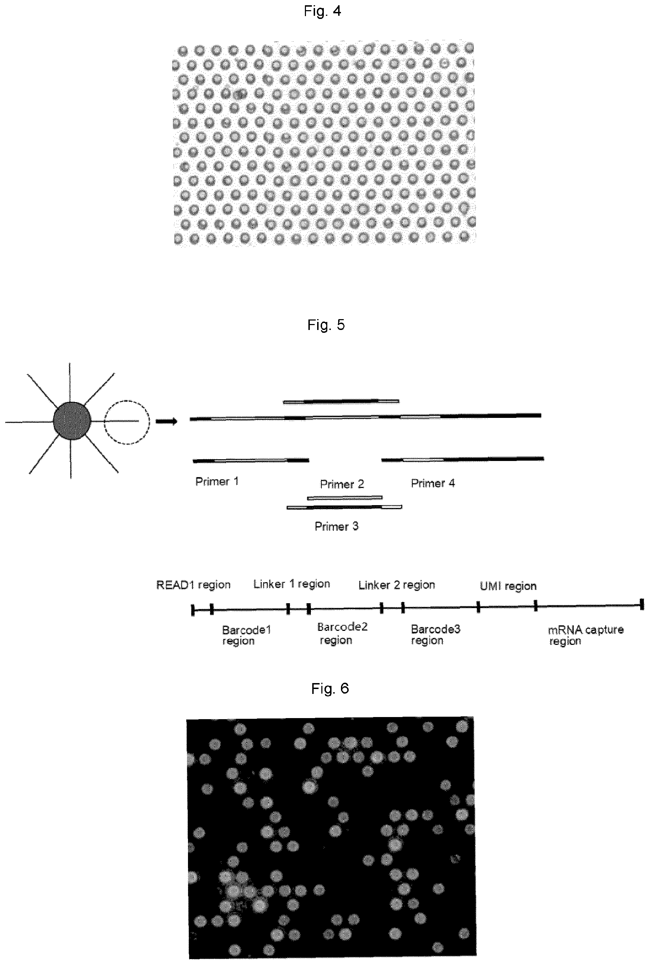

A schematic diagram of a sequence structure of the silicon dioxide microspheres coded with long sequences synthesized by a ligation method is shown in Fig. 5.

It is clear to those skilled in the art that nucleic acids include deoxyribonucleic acid and ribonucleic acid, and are one of the most fundamental substances of life, and play a vital role in a wide range of life activities of organisms, such as growth, development, mutation, inflammation, cancer, etc. Nucleic acid molecules are closely related to and play an important role in the development of various diseases that affect human health. Therefore, the development of accurate and efficient methods for sensitive and accurate detection is of great significance for in-depth investigation of functional regulation of nucleic acids, drug screening, as well as early detection, clinical diagnosis and treatment, and prognostic evaluation of related diseases.

Silicon dioxide is a typical inorganic powder material with a large specific surface area and good chemical stability. Monodisperse silicon dioxide microspheres are simple in preparation process and good in biocompatibility, such that silicon dioxide microspheres with different sizes and different surface modifications may be applied to different fields such as information, biology, medicine, etc.

Due to limitation in mechanism of action, traditional sequencing methods may lead to omission of important information, however, nucleic acids contain more biological information and a large amount of data, and thus the use of oligonucleotide strands coded by long sequences modified on a substrate, which is then used for DNA sequencing, is of great significance in collection of nucleic acid information.

Therefore, in a third preferred embodiment of the present invention, a method for synthesizing silicon dioxide microspheres coded with long DNA sequences is further provided. The method includes connecting carboxylated silicon dioxide and a sequence carrying amino through a condensation reaction, then adding a complementary sequence of a subsequent barcode sequence, and obtaining the silicon dioxide microspheres coded with long sequences through primer annealing and a polymerase polymerization reaction, so as to further apply the obtained microspheres to DNA chip decoding.

The referred embodiment uses the following technical solutions.
1. Primer design: a coding primer sequence is designed.
2. Activation of carboxylated silicon dioxide microspheres: the silicon dioxide microspheres carrying carboxyl are activated by EDC and NHS, so as to obtain well-dispersed silicon dioxide microspheres with exposed carboxyl on the surface.
3. Connection reaction: the carboxylated silicon dioxide microspheres and an oligonucleotide strand with the end modified with amino are connected together through the condensation reaction so as to obtain the silicon dioxide microspheres with different sequences.
4. Barcode sequence synthesis: subsequent several sequences are successively connected to the silicon dioxide microspheres of step 3 through a polymerization reaction, so as to finally obtain the silicon dioxide microspheres coded with long sequences.

Patterns of gene expression in a spatial original position in tissue are important for understanding the types and functions of cells therein. In recent years, a spatial transcriptome technology develops rapidly and is widely used in different fields such as tumors, diseases, nervous systems and organ development.

Carriers now used to manufacture biological chips generally have active groups that may be chemically reacted so as to be used to couple biomolecules. The type of the carriers mainly includes glass slides, silicon wafers, nitrocellulose membranes, nylon membranes, etc. A spatial transcriptome chip in the prior art uses a glass slide made of glass, and such long-lasting carrier is resistant to high temperature and has high ionic strength, and is also non-wetting, such that a hybridization volume is minimized. In addition, a hydrophobic surface overcomes the disadvantage of easy spreading of samples, and the density of sample points is increased. A low fluorescence signal also does not cause strong background interference. The biological chip is to immobilize a spatially-analyzed capture probe onto the glass slide, but the biological chip has the disadvantage of low resolution of sample analysis. Therefore, how to improve the resolution of sample analysis and reduce the cost of consumables so as to meet the needs of scientists for subcellular structure analysis is an urgent problem to be solved.

Therefore, in a fourth preferred embodiment of the present invention, a chip with improved structure is further provided. A spatial transcriptome chip of the present invention is described with reference to Figs. 9-14. The spatial transcriptome chip includes a substrate 101, a plurality of rectangular microporous regions 201 are formed on the substrate 101, a plurality of sub-rectangular microporous regions 301 are formed in the rectangular microporous regions 201, a plurality of microporous structures 70 are uniformly distributed in each sub-rectangular microporous region 301, the microporous structure is configured to place coded microspheres.

Further, the substrate 101 uses a glass substrate, the glass substrate is etched to form the microporous structures 70, and the microporous structures 70 are all circular holes. Furthermore, the sub-rectangular microporous region 301 is formed from the uniformly-distributed microporous structures 70, one rectangular microporous region 201 is constructed from the plurality of sub-rectangular microporous regions 301, and the plurality of rectangular microporous regions 201 are constructed on the glass substrate.

Specifically, the length and width of the rectangular microporous region 201 all range from 5 mm to 20 mm. The length and width of the sub-rectangular microporous region 301 all range from 100 µm to 300 µm. The diameter of the microporous structure 70 ranges from 1 µm to 10 µm, and the minimum value of a distance between central points of the adjacent microporous structures 70 ranges from 1 µm and 2 µm.

As shown in Fig. 9, this preferred embodiment provides a size design of the spatial transcriptome chip. The length, width, and thickness of the glass substrate respectively are 75 mm, 25 mm and 1 mm, respectively. Eight rectangular microporous regions 201 are arranged on the glass substrate, and are arranged in four rows in a length direction, and in two columns in a width direction. The distance between the rectangular microporous region 201 and the top of the glass substrate is 8 mm, and the distance from the side of the glass substrate is 3.3 mm. The distance between the two columns of the rectangular microporous regions 201 is 4 mm, and the distance between the rectangular microporous regions 201 between the adjacent rows is 5 mm. The length and width of each rectangular microporous region 201 respectively are 7.2 mm and 7.2 mm. The aperture of the microporous structure 70 etched on the substrate 101 is approximately 2.5 um, which is equivalent to increasing the resolution of the current mainstream spatial transcriptome by more than 20 times and may be used for HE staining and gene expression experiments on the glass slide later.

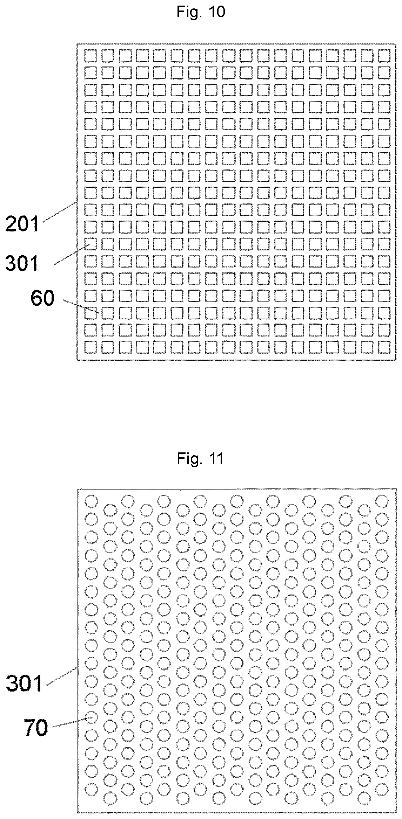

According to the spatial transcriptome chip provided in this embodiment, the plurality of rectangular microporous regions 201 are constructed on the substrate 101, the plurality of sub-rectangular microporous regions 301 are also formed in each rectangular microporous region 201, and the microporous structures 70 (as shown in Fig. 11) are uniformly distributed in each sub-rectangular microporous region 301. The resolution of a spatial transcriptome is improved through the microporous structures 70 to realize high sensitivity detection, such that requirements of scientists for subcellular structure analysis are met. In addition, using the glass substrate, the microporous structures 70 may be manufactured through an etching technology, such that a manufacturing process is simple, and consumable costs are reduced.

According to the spatial transcriptome chip provided in a preferred embodiment of the present invention, as shown in Fig. 10, a gap 60 is formed between the adjacent sub-rectangular microporous regions 301, and the size of each gap 60 is the same. Specifically, the width of the gap 60 ranges from 2 µm to 20 µm. It is to be noted that, the width of the gap refers to the distance between the central points of the two microporous structures 70. In the prior art, when a scanning device scans the microporous structures 70 on the substrate 101, scanning images need to be spliced with a software algorithm, the scanning images at the moments before and after need to be spliced during splicing, but due to numerous microporous structures 70, splicing errors occur from time to time during splicing. In this embodiment, a plurality of gaps 60 are provided inside the rectangular microporous region 201, and then a plurality of small-sized sub-rectangular microporous regions 301 are divided, such that images in different regions are identified by the scanning device and software, correction of image collection at a later stage is conveniently achieved, and positioning errors of chip coding information are avoided, thereby improve splicing accuracy. It is to be understood that, the width of the gap 60 may be processed and manufactured according to a specific scanning device and a software algorithm, and the width of the gap 60 is not limited in the present invention.

According to the spatial transcriptome chip provided in the preferred embodiment of the present invention, at least one of the sub-rectangular microporous regions 301 located on four right angles of the rectangular microporous region 201 is provided with a marker. During image splicing and post processing, directions of the images all need to be adjusted. Therefore, in this embodiment, the markers are provided in the sub-rectangular microporous regions 301 located at right angles of the rectangular microporous region 201, such that orientations are easy to distinguish, and rotation directions and angles of the images are identified, thereby improving the accuracy of image processing. It may be understood that, the sub-rectangular microporous regions 301 in different rectangular microporous regions 201 may use different forms of markers, so as to achieve convenient identification.

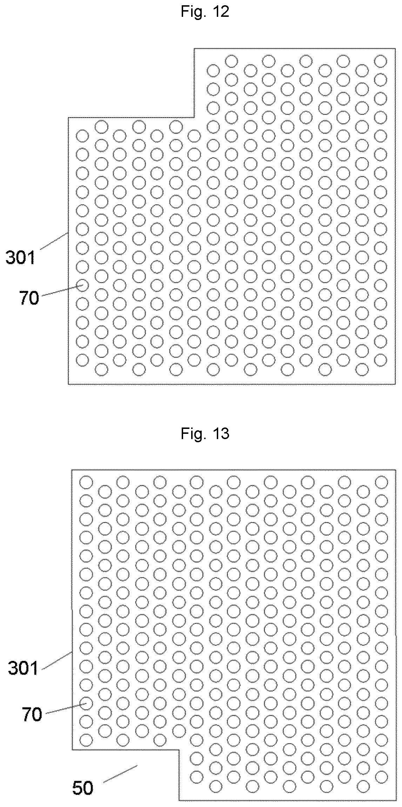

Further, notches 50 are formed in the sub-rectangular microporous regions 301 located in any three positions of the four right angles of the rectangular microporous region 201, so as to form markers; and the sub-rectangular microporous region 301 on the other right angle is not provided with the notch 50, and is a normal rectangular region. Specifically, the notch 50 is located in any one of the right angles of the sub-rectangular microporous region 301. Further, the shape of the notch 50 may be a rectangle shown in Figs. 12 and 13, and may also be a triangle shown in Fig. 14. In this embodiment, the right angle position of the sub-rectangular microporous region 301 is used to be etched to form the notch, so as to form the marker, such that orientations are easy to distinguish, and rotation directions and angles of the images are identified.

As described above, patterns of gene expression in a spatial original position in tissue are important for understanding the types and functions of cells therein. In recent years, a spatial transcriptome technology rapidly develops and is widely used in different fields such as tumors, diseases, nervous systems, and organ development. Carriers now used to manufacture biological chips generally have active groups that may be chemically reacted so as to be used to couple biomolecules. The type of the carriers mainly includes glass slides, silicon wafers, nitrocellulose membranes, nylon membranes, etc.

There are two kinds of spatial transcriptome chips in the prior art.
1. A chip made of glass, and such long-lasting carrier is resistant to high temperature and high ionic strength, and is also non-wetting, such that a hybridization volume is minimized. In addition, a hydrophobic surface overcomes the disadvantage of easy spreading of samples, and the density of sample points is increased. A low fluorescence signal also does not cause strong background interference. The biological chip is to immobilize a spatially-analyzed capture probe onto the glass slide, but the biological chip has the disadvantage of low resolution of sample analysis, which cannot meet the needs of scientists for subcellular structure analysis.
2. A silicon wafer is used as a substrate of a spatial transcriptome biological chip. Such chip made of the silicon wafer, developed based on gene sequencing, may achieve subcellular-level spatial transcriptome analysis. However, bright-field microscopic imaging such as HE staining, toluidine blue staining, and Masson staining cannot be performed on a non-transparent substrate, imaging of tissue can only be implemented by means of fluorescence, or bright-field imaging is performed using a temporary chip, resulting in final gene expression results that cannot be well analyzed in conjunction with microscope collection results.

Therefore, according to a fifth aspect of the present invention, another spatial transcriptome biological chip with an improved structure is further provided. A spatial transcriptome biological chip of this preferred embodiment is described with reference to Figs. 15-24. The spatial transcriptome biological chip includes a transparent substrate 10. The transparent substrate 10 is subjected to photolithography and etching to form a microporous region 20. The microporous structure 70 is formed within the microporous region 20. The microporous structure 70 is configured to place coded microspheres with primers.

Further, the transparent substrate 10 in this embodiment may use glass, quartz, plastic, or a transparent conductive coating. The transparent conductive coating may use one of magnesium chloride, gallium arsenide, etc. The transparent substrate 10 is processed by means of photolithography and etching to form the microporous region 20. The microporous structure 70 in the microporous region 20 can place the coded microspheres with primers.

The microporous region 20 in this embodiment may be a microporous region 20 that is formed by uniformly spreading the microporous structures 70 integrally, and may also form sub-regions 30 with different shapes (which are specifically described with examples in the following embodiments). The images may be conveniently spliced and identified by different scanning software according to the sub-regions 30 with different shapes. The resolution of a spatial transcriptome is improved through the microporous structures 70 to realize high sensitivity detection and meet the requirements of scientists for subcellular structure analysis. In addition, using the glass substrate, the microporous structures 70 may be manufactured with an etching technology, with a simple manufacturing process and reduced consumable costs.

The spatial transcriptome biological chip in this embodiment may be subjected to bright-field microscopic imaging such as HE staining, toluidine blue staining, and Masson staining, and comparative analysis may be performed by combining a gene expression result and a bright-field staining result.

The spatial transcriptome biological chip provided in this preferred embodiment, utilizes the transparent substrate. The microporous structures are manufactured through the photolithography and etching technology, and the microporous regions are formed. The biological chip is simple in manufacturing process, and consumable costs are reduced. Furthermore, through bright-field imaging, an analysis effect of the spatial transcriptome is greatly improved by combining with a gene expression result. In addition, the microporous structures of the chip are high in resolution, enabling the subcellular-level spatial transcriptome data analysis.

In one of preferred embodiments, at least one sub-region 30 is formed in the microporous region 20, and the sub-region 30 is a circular region or a polygonal region. It may be understood that, the sub-region 30 in this embodiment is also constituted by the plurality of microporous structures 70, and a plurality of sub-regions 30 form one entire microporous region 20 according to a specific arrangement. For example, as shown in Fig. 15, the sub-region 30 is a large rectangle. As shown in Fig. 16, the sub-regions 30 are arranged in small rectangles in plurality of rows and columns. As shown in Fig. 17, the sub-regions 30 are arranged in two circles. As shown in Fig. 18, the sub-regions 30 are arranged in four hexagon shapes. It may be understood that, there may be one sub-region 30 shown in Fig. 1, and there may also be a plurality of sub-regions shown in Figs. 16, 17 and 18. The shape of the sub-region 30 may be not only a circle, but also a polygon such as a triangle, a rectangle, a hexagon, and the like, and examples are not given here.

In one of preferred embodiments, a plurality of micro-units 40 are formed in the sub-region 30. The adjacent micro-units 40 are arranged at intervals, and the micro-units 40 are repeatedly arranged in circles or polygons. In this embodiment, a plurality of smaller micro-units 40 are also formed in the sub-region 30. The micro-units 40 in the same sub-region 30 are uniformly arranged in circles or polygons. For example, as shown in Fig. 19, the micro-units 40 may be uniformly arranged in rectangles, or as shown in Fig. 20, the micro-units may be distributed in hexagons. It may be understood that, as shown in Fig. 21, the micro-unit 40 is also constituted by the plurality of microporous structures 70, and the plurality of micro-units 40 form one sub-region 30 according to a specific arrangement. In the prior art, when a scanning device scans the microporous structures 70 on the substrate, scanning images need to be spliced with a software algorithm, the scanning images at the moments before and after need to be spliced during splicing, but due to numerous microporous structures 70, splicing errors often occur during splicing. In this embodiment, a plurality of gaps 60 are provided inside the sub-region 30, and then the sub-region 30 is divided into a plurality of small-sized micro-units 40, facilitating the identification of images in different regions with the scanning device and software, simplifying the image collection at a later stage, avoiding the misalignment of chip encoding information, and thereby improving the splicing accuracy.

Further, if the sub-region 30 is a square, a size of the sub-region 30 ranges from 9mm² to 1875mm². Definitely, the size of the sub-region may also be correspondingly adjusted according to different shapes of the sub-regions 30 and different experiment requirements. The distance between the micro-units 40 is between 0 µm and 40 µm, and the same is true for the micro-units 40.

In one of preferred embodiments, a notch 50 is formed at an edge and/or a corner of the micro-unit 40, so as to form an azimuth marker. During image splicing and post processing, the orientation of images needs to be adjusted. Therefore, in this embodiment, the azimuth markers are provided at the edges and/or corner positions of the micro-units 40 located at edges and/or corner positions of the sub-region 30, such that orientations are easy to distinguish, and rotation directions and angles of the images are identified, thereby improving the accuracy of image processing. Specifically, the micro-unit 40 provided with the notch 50 in this embodiment refers to the micro-unit 40 located at the edge and/or a corner position of the sub-region 30. The shape of the notch 50 may be a rectangle, a triangle, etc. The notch 50 is formed by cutting a portion of the micro-unit 40, so as to form the azimuth marker, enabling that orientations are easy to distinguish, and rotation directions and angles of the images are identified. Also, because the shape of the micro-unit 40 may be a circle or a polygon, the notch 50 may be in an edge position of the micro-unit 40 shown in Fig. 7; or the notch 50 may be in the position of the corner of the micro-unit 40 shown in Fig. 22. Fig. 23 is a schematic structural diagram of a micro-unit 40 without forming a notch 50.

In one of preferred embodiments, a diameter of the microporous structure 70 ranges from 0.1 µm to 10 µm, and a distance between central points of adjacent two micropores is between 0.1 µm and 20 µm. In this embodiment, whether the microporous region 20 is formed by spreading the microporous structures 70 or is formed by the sub-regions 30 or the micro-units 40, the microporous structures 70 are all formed in the microporous region. The microporous structures 70 may be in the form of circular micropores, with the diameter being between 0.1 µm to 10 µm. The distance between the central points of the adjacent two micropores is between 0.1 µm and 20 µm. Definitely, according to different requirements, the sizes of the diameters of the micropores and the size of the distance between the central points of the micropores may also be adjusted correspondingly.

In one of preferred embodiments, a long side of the transparent substrate 10 ranges from 10 to 100 mm, and a short side ranges from 5 to 50 mm. For example, the sizes of the length, width, and height of the transparent substrate 10 may use 75 mm*25 mm*1 mm. Definitely, the size of the transparent substrate 10 may be correspondingly designed according to actual requirements.

In one of preferred embodiments, as shown in Fig. 24, the microporous structure 70 includes an expansion portion 71 located on a surface of the transparent substrate 10 and a shrinkage portion 72 located in the transparent substrate 10. The expansion portion 71 and the shrinkage portion 72 are interconnected along a depth direction of the transparent substrate 10, so as to form the microporous structure 70 on the transparent substrate 10. In this embodiment, the microporous structure 70 is a funnel-shaped deep cone structure in the depth direction. The diameter of the expansion portion 71 provided on the surface of the transparent substrate 10 is greater than the diameter of the shrinkage portion 72 provided within the transparent substrate 10, that is, the diameter of the microporous structure 70 gradually reduces from the surface to the inside of the transparent substrate 10. By processing the microporous structure 70, the diameter of the expansion portion 71 is larger, such that the coded microspheres are conveniently positioned and placed more easily. Further, an oblique angle of the deep cone structure is between 0° and 60°, that is, an included angle between a connection line between the expansion portion 71 and the shrinkage portion 72 and the depth direction of the transparent substrate 10 is between 0° and 60°. Definitely, the angle may be processed and adjusted according to actual requirements.

In one of preferred embodiments, as shown in Figs. 23 and 24, the transparent substrate 10 has a first surface 11 and a second surface 12. The first surface 11 and the second surface 12 are subjected to photolithography and etching to form the microporous regions 70, respectively. The first surface 11 and the second surface 12 are two opposite surfaces of the transparent substrate 10. In this embodiment, by processing the microporous regions on positive and negative surfaces (i.e., the first surface 11 and the second surface 12) of the transparent substrate 10, when the coded microspheres are placed on the first surface 11 (i.e., the positive surface), other processes may be performed on the second surface 12 (i.e., a bottom surface), for example, pre-processing such as cleaning or post-processing is performed on the microporous regions, such that overall working efficiency can be improved.

This preferred embodiment provides a size design of the spatial transcriptome biological chip. The length, width, and thickness of the glass substrate respectively are 75 mm, 25 mm, and 1 mm. Eight rectangular sub-regions 30 are arranged on the glass substrate, and are arranged in four rows in a length direction, and in two columns in a width direction. The distance between the rectangular sub-regions 30 and the top of the glass substrate is 8 mm, and the distance from the side of the glass substrate is 3.3 mm. The distance between the two columns of the rectangular sub-regions 30 is 4 mm, and the distance between the rectangular sub-regions 30 between the adjacent rows is 5 mm. The length and width of each rectangular sub-region 30 respectively are 7.2 mm and 7.2 mm. The aperture of the microporous structure 70 etched on the transparent substrate 10 is approximately 2.5 um, which is equivalent to increasing the resolution of the current mainstream spatial transcriptome by more than 20 times, and may be used for HE staining and gene expression experiments on the glass slide later.

A human body, as an organic whole, consists of a variety of tissues, and cells in the tissues vary in type, time, and space, making the study of spatial specificity particularly important. Spatial omics may preserve the spatial structural integrity of a sample through tissue sectioning, thus obtaining gene expression in different regions. Therefore, spatial analysis at a subcellular level is an important tool for the study of tissue and organ functions and vital activities of living organisms.

A biological chip may be manufactured by synthesizing different sequence-modified microspheres. Each microsphere has a corresponding probe. The microspheres are embedded in well positions on a surface of a glass plate after being uniformly mixed, and then the chip is decoded through different fluorescence probes, so as to obtain a decoded chip. The decoded biological chip may capture mRNA in a tissue section in situ and perform subsequent sequencing steps, so as to obtain transcriptome sequence information having tissue spatial position information. The sequencing method may implement tissue spatial transcriptome sequencing at multicellular, unicellular, and subcellular resolutions.

Traditional biological chips obtain less information obtained by a one-step hybridization reaction, and are low in decoding efficiency, so developing a three-color fluorescence decoding method facilitates better study of spatial position information of tissues at the subcellular level.

Therefore, according to a sixth aspect of the present invention, in some preferred embodiments, a three-color fluorescence decoding method based on a subcellular-level spatial chip is provided. The biological chip involved in the method is prepared by embedding silicon dioxide microspheres modified by different sequences to be decoded into a glass substrate with well positions, and then performing, for a plurality of times, a hybridization reaction on probes marked with different fluorescence dyes and the chip, so as to obtain the decoded biological chip. In the method, more fluorescence dyes may be introduced to reduce decoding cycles.

This referred embodiment uses the following technical solutions.
(1) Design of a primer sequence and a probe sequence: a coding primer sequence and a decoding probe sequence correspondingly having different fluorescent tags are designed.
(2) Probe hybridization reaction: decoding probes having different fluorescent tags of each round are mixed, then a hybridization reaction is performed with the biological chip, and then corresponding fluorescence signals are detected.
(3) DNA denaturation and cleaning: the DNA hybridized chip is denatured through sodium hydroxide, and the chip is cleaned for a next round of the hybridization reaction.
(4) Chip decoding: a microsphere sequence is decoded according to each round of fluorescence signals of each well on the chip.

The beneficial effects of the present application are further described in detail below with reference to specific embodiments.

It is to be noted that, the following embodiments are used to illustrate the present invention, but not to limit the scope of the present invention. If not specifically indicated, the technical means used in the embodiments are conventional means known to those skilled in the art, and raw materials used are commercially available. If not specifically indicated, all the embodiments are in accordance with conventional experimental conditions, such as the Sambrook et al. Molecular Cloning Laboratory Manual (Sambrook J & Russell DW, Molecular Cloning: a Laboratory Manual, 2001), or in accordance with the conditions recommended by manufacturer instructions.

Terms involved in the following embodiments:
EDC: 1-(3-(dimethylaminopropyl)-3-ethylcarbodiimide hydrochloride
NHS: N-hydroxysuccinimide
MES: 2-morpholineethanesulfonic acid

### Embodiment 1 Preparation method of a microsphere chip

1. Surface cleaning of a chip
   (1) A Piranha Solution (concentrated sulfuric acid: hydrogen peroxide solution = 7:3, a volume ratio) was prepared in a fume hood, and an etched silicon dioxide chip (a silicon dioxide glass slide etched with micropores) was added into the Piranha Solution to soak for 30 min while hot.
   (2) The chip washed by the piranha water was put into a beaker with ultrapure water, washing was performed with the ultrapure water for three times, and drying was performed with clean nitrogen gas.
   (3) The chip was washed with analytical pure anhydrous ethanol for three times, and drying was performed with the clean nitrogen gas for later use.
2. Chip coating processing
   (1) A spin-coater was used to spread an UV adhesive in a chip target region such that a thin layer of the adhesive was attached to the entire chip region.
   (2) The chip was placed under an ultraviolet lamp, irradiation was performed for 4 min to cure the UV adhesive.
3. Microsphere filling
   (1) Centrifugation was performed on 20 µL of synthesized coded silicon dioxide microspheres, and supernatant was discarded.
   (2) 200 µL of ultrapure water was added for resuspending, supernatant was discarded after centrifugation, and the operations were repeated for 2 times.
   (3) 150 µL of a 5‰ ultraviolet curing adhesive solution was added to resuspend the microspheres, and after counting was performed with a hemocytometer, the microspheres were diluted again to 2×10⁵/µL.
   (4) The chip with coating processed was mounted on a spatial transcription chip clamping apparatus.
   (5) 150 µL of a well-mixed microsphere suspension was taken and added to the chip target region, and standing was performed for 4 min at room temperature.
   (6) The assembled microspheres and the chip were placed on a plate centrifuge, centrifugation was performed for 20-30s, supernatant was gently well mixed, and then centrifugation was performed for 20-30s at 2000 rpm.
4. Chip cleaning
   (1) A pipette was used to remove the suspension of a target well position.
   (2) The self-assembled chip was taken, and pulled and washed for 5 times in the ultrapure water, spin-drying was performed, and then standing and airing were performed at room temperature.
   (3) The chip was swept for a plurality of times with a cleaning small brush, until no visible microsphere residue remains in the target region.
5. Chip quality control
   (1) The self-assembled chip was well marked.
   (2) A microscope was used to scan the target region, scanning was performed with a 40x object lens, and then drop hole efficiency was analyzed.

An effect diagram of the silicon dioxide chip of the present invention before assembly was shown in Fig. 1.

An effect diagram of the silicon dioxide chip of the present invention before assembly was shown in Fig. 2. An effect diagram of the silicon dioxide chip and the microspheres after self-assembly was shown in Fig. 3.

### 6. High temperature quality control

The chip was placed on a module of a 95°C metal bath and incubated for 30 min, then washing was performed for three times with the ultrapure water, the chip was scanned in a scanner, a quality control chart before heat incubation was compared, and results showed that the structure of the chip was not affected by high temperature processing, and the drop hole rate was still higher than 99%.

The chip processing for 30 min at 95°C was shown in Fig. 4.

### Embodiment 2 Optimization of ultraviolet adhesive selection

Three types of adhesives were respectively tested: Feifanli 3217 UV glue, Kingstar UV glue, And Ergo 1309, a Swiss imported Ab adhesive. Feiranli 3217 was finally selected.
1. Surface cleaning of a chip
   (1) A Piranha Solution (concentrated sulfuric acid: hydrogen peroxide at a 7:3 volume ratio) was prepared in a fume hood. An etched silicon dioxide chip (a silicon dioxide glass slide etched with micropores) was added into the Piranha Solution and soaked for 30 minutes while hot.
   (2) The chip, after being washed by the Piranha Solution, was put into a beaker with ultrapure water, It was rinsed three times with ultrapure water and then dried with clean nitrogen gas.
   (3) The chip was rinsed three times with analytical pure anhydrous ethanol, and then dried with the clean nitrogen gas for later use.
2. Chip coating processing
   (1) A plate centrifuge was used to spread the Feifanli 3217 UV glue, the Kingstar UV glue, and the ergo1309 Swiss imported AB adhesive separately in the chip target region, ensuring that a thin layer of the adhesive was attached to the entire chip region.
   (2) The chip was placed under an ultraviolet lamp and irradiated for 4 min to cure the UV adhesive.
3. Microsphere filling
   (1) Centrifugation was performed on 20 µL of synthesized coded silicon dioxide microspheres, and the supernatant was discarded.
   (2) 200 µL of ultrapure water was added to resuspend the microspheres. after centrifugation, the supernatant was discarded, and the operations were repeated for 2 times.
   (3) 150 µL of ultrapure water (containing a 5‰ ultraviolet curing adhesive) was added to resuspend the microspheres. After counting with a hemocytometer, the microspheres were diluted again to 2×10⁵-3×10⁵/µL.
   (4) The chip with the coating processed was mounted on a spatial transcription chip clamping apparatus.
   (5) 150 µL of a well-mixed microsphere suspension was taken and added to the chip's target region. It was allowed to stand for 4 min at room temperature.
   (6) The assembled microspheres and the chip were placed in a plate centrifuge. Centrifugation was performed for 20-30 seconds, after which the supernatant was gently mixed. Then centrifugation was performed for 20-30 seconds at 2000 rpm.
4. Chip cleaning
   (1) The suspension from the target well position was removed with a pipette.
   (2) The self-assembled chip was washed 5 times with ultrapure water, spin-dried, and then allowed to stand and air at room temperature.
   (3) The chip was brushed multiple times with a cleaning small brush until no visible microsphere residue remains in the target region.
5. Chip quality control
   (1) The self-assembled chip was carefully labeled.
   (2) A microscope was used to scan the target region with a 40x object lens, and then drop hole efficiency was analyzed.

The optimization test results for ultraviolet adhesive selection are shown in Table 1. Experimental results indicated that the Feifanli 3217 UV glue exhibited the best performance.

**Table 1**

| Ultraviolet adhesive manufacturer | Drop hole rate |
|---|---|
| Feifanli 3217 UV glue | 99% |
| Kingstar UV glue | 83% |
| Ergo1309 Swiss imported AB adhesive | 50% |

### Embodiment 3 Optimization of microsphere spin-coating concentration

A silicon dioxide microsphere solution was prepared using ultrapure water, three microsphere spin-coating concentrations were tested: 10 W/µL, 20 W/µL, and 30 W/µL. After evaluation, a concentration of 20 W/µL was finally determined to be the most effective for spin-coating.
1. Surface cleaning of a chip
   (1) A Piranha Solution (concentrated sulfuric acid: hydrogen peroxide solution at a 7:3 volume ratio) was prepared in a fume hood. An etched silicon dioxide chip (a silicon dioxide glass slide etched with micropores) was added into the Piranha Solution and soaked for 30 minutes while hot.
   (2) The chip, after being washed by the Piranha Solution, was put into a beaker with ultrapure water, It was rinsed three times with ultrapure water and then dried with clean nitrogen gas.
   (3) The chip was rinsed three times with analytical pure anhydrous ethanol, and then dried with the clean nitrogen gas for later use.
2. Chip coating processing
   (1) A plate centrifuge was used to spread the Feifanli 3217 UV glue in a chip target region such that a thin layer of the adhesive was attached to the entire chip region.
   (2) The chip was placed under an ultraviolet lamp and irradiated for 4 min to cure the UV adhesive.
3. Microsphere filling
   (1) Centrifugation was performed on 20 µL of synthesized coded silicon dioxide microspheres, and supernatant was discarded.
   (2) 200 µL of ultrapure water was added for resuspending, supernatant was discarded after centrifugation, and the operations were repeated for 2 times.
   (3) 150 µL of the ultrapure water was added to resuspend the microspheres, and after counting was performed with a hemocytometer, the microspheres were diluted again to three concentrations of 10 W/µL, 20 W/µL, and 30 W/µL.
   (4) The chip with coating processed was mounted on a spatial transcription chip clamping apparatus.
   (5) 150 µL of a well-mixed microsphere suspension was taken and added to the chip target region, and standing was performed for 4 min at room temperature.
   (6) The assembled microspheres and the chip were placed on a plate centrifuge, centrifugation was performed for 20-30s, supernatant was gently well mixed, and then centrifugation was performed for 20-30s at 2000 rpm.
4. Chip cleaning
   (1) A pipette was used to remove the suspension of a target well position.
   (2) The self-assembled chip was washed 5 times with ultrapure water, spin-dried, and then allowed to stand and air at room temperature.
   (3) The chip was brushed multiple times with a cleaning small brush, until no visible microsphere residue remains in the target region.
5. Chip quality control
   (1) The self-assembled chip was well labeled.
   (2) A microscope was used to scan the target region, scanning was performed with a 40x object lens, and then drop hole efficiency was analyzed.

Optimization results of microsphere spin-coating concentrations were shown in Table 2. Experimental results showed that 20 W/µL of the microsphere suspension was the optimal concentration.

**Table 2:**

| Microsphere spin-coating concentration | Drop hole rate |
|---|---|
| 10W/µL | 90% |
| 20W/µL | 99% |
| 30W/µL | 95% |

### Embodiment 4 Optimization of microsphere resuspension buffer

Microsphere resuspension buffers were tested using the following reagents:
a, Ultrapure water.
b, Three DMSO solutions with concentrations of 5%, 10%, and 20% respectively.
c, Ultraviolet curing adhesive solutions with concentrations of 2.5‰, 5‰, and 10‰, respectively.

After experimentation, the 5‰ ultraviolet curing adhesive solution was finally determined to be the optimal microsphere resuspension buffer.
1. Surface cleaning of a chip
   (1) A Piranha Solution (concentrated sulfuric acid:hydrogen peroxide solution = 7:3, a volume ratio) was prepared in a fume hood, and an etched silicon dioxide chip (a silicon dioxide glass slide etched with micropores) was added into the Piranha Solution to soak for 30 min while hot.
   (2) The chip washed by the piranha water was put into a beaker with ultrapure water, washing was performed with the ultrapure water for three times, and drying was performed with clean nitrogen gas.
   (3) The chip was washed with analytical pure anhydrous ethanol for three times, and drying was performed with the clean nitrogen gas for later use.
2. Chip coating processing
   (1) A plate centrifuge was used to spread the Feifanli 3217 UV glue in a chip target region such that a thin layer of the adhesive was attached to the entire chip region.
   (2) The chip was placed under an ultraviolet lamp, irradiation was performed for 4 min to cure the UV adhesive.
3. Microsphere filling
   (1) Centrifugation was performed on 20 µL of synthesized coded silicon dioxide microspheres, and supernatant was discarded.
   (2) 200 µL of ultrapure water was added for resuspending, supernatant was discarded after centrifugation, and the operations were repeated for 2 times.
   (3) 7 test buffers were added to resuspend the microspheres, i.e., a, the ultrapure water; b, the DMSO solutions with the concentrations being respectively 5, 10, and 20%; and c, the ultraviolet curing adhesive solutions with the concentrations being respectively 2.5, 5, and 10‰; and the microspheres were diluted again to the concentration being 20 W/µL.
   (4) The chip with coating processed was mounted on a spatial transcription chip clamping apparatus.
   (5) 150 µL of a well-mixed microsphere suspension was taken and added to the chip target region, and standing was performed for 4 min at room temperature.
   (6) The assembled microspheres and the chip were placed on a plate centrifuge, centrifugation was performed for 20-30s, supernatant was gently well mixed, and then centrifugation was performed for 20-30s at 2000 rpm.
4. Chip cleaning
   (1) A pipette was used to remove the suspension of a target well position.
   (2) The self-assembled chip was taken, and pulled and washed for 5 times in the ultrapure water, spin-drying was performed, and then standing and airing were performed at room temperature.
   (3) The chip was swept for a plurality of times with a cleaning small brush, until no visible microsphere residue remains in the target region.
5. Chip quality control
   (1) The self-assembled chip was well marked.
   (2) A microscope was used to scan the target region, scanning was performed with a 40x object lens, and then drop hole efficiency was analyzed.

Optimization results of the microsphere resuspension buffers were shown in Table 3.

Experimental results showed that the 5‰ ultraviolet curing adhesive solution was the optimal microsphere resuspension buffer.

**Table 3**

| Microsphere resuspension buffer | Drop hole rate |
|---|---|
| Ultrapure water | 50% |
| 5% DMSO solution | 40% |
| 10% DMSO solution | 59% |
| 20% DMSO solution | 75% |
| 2.5‰ ultraviolet curing adhesive solution | 89% |
| 5‰ ultraviolet curing adhesive solution | 99% |
| 10‰ ultraviolet curing adhesive solution | 93% |

Through the microsphere chip preparation method invention above, a high drop hole rate was achieved, and the microspheres were more resistant to fall off, and were more secure compared to a method of dropping holes directly by physical squeezing.

### Embodiment 5 Method for synthesizing Barcode silicon dioxide microspheres by a ligation method

This embodiment provided a method for synthesizing silicon dioxide microspheres coded with long sequences by a liagtion method, which included using the silicon dioxide microspheres as carriers, connecting the microspheres and a first oligonucleotide sequence through a condensation reaction, then continuously extending subsequent second and third primers through T4 ligase, and finally obtaining various microspheres coded with long sequences.

The method included the following steps.
1. Activation of carboxylated silicon dioxide microspheres.
1.1 A silicon dioxide microsphere stock solution was diluted by 200 times, and then accurate counting (about 300000/µL) was performed under a microscope.
1.2 MES(100 mg), EDC(20 mg), and NHS(15 mg) were weighed, the MES was prepared to 0.5M, 16 mL of the prepared MES was sucked to dissolve the EDC and the NHS, then the mixed solution was used to resuspend counted microspheres, the mixed solution was dispensed into 10 1.5mL EP tubes, with 400 µL for each tube, and a reaction was performed overnight at room temperature on a metal oscillator (1000 rpm) for activation.

2. Barcode 1 condensation reaction
The activated silicon dioxide microspheres were uniformly dispensed in a 96-well plate, with 20 µL in each well. Then 5 µL of amino-modified oligonucleotide (primer 1 dissolved in 0.1M MES to a final concentration of 60 µM) was added to each well. Mixing was performed through oscillation, and the shaking speed of the metal oscillator was set to 300-2000 rpm and then oscillating at room temperature for reaction overnight. After the reaction ended, the microspheres were collected into a 50mL centrifuge tube and washed with a PBS containing 0.001-0.03%v/v tween-20. The supernatant was carefully removed after centrifugation, then the microspheres were washed twice in TE Buffer and were resuspended in enzyme-free water.
3. Barcode 2 primer annealing reaction (100µL system)
Primer 2 and 3 were mixed in a 1:1 molar ratio, and then a 5× annealing buffer was added for the annealing reaction. The PCR procedure settings included: starting at 95°C and gradually cooling at 15°C, performing annealing at 10°C intervals every 3 minutes, and finally obtaining a Barcode 2 primer (annealing product), with a primer concentration of 100µM.
A formula of the 5× annealing buffer was as follows.

| Component | amount |
|---|---|
| Tris-HCl solution (1M) | 30µL |
| EDTA (0.5M) | 15µL |
| NaCl(2M) | 100µL |
| Double distilled water | Make up to a total volume of 1000 µL |

4. Barcode 2 connection reaction
The condensed microspheres were uniformly dispensed into a 96-well plate. Then, 5µL of a 5×T4 connection buffer, 5µL of 500U/µL T4 ligase, and 5µL of the Barcode 2 primer (100µM) were added, bringing the total volume to 50µL with water. The reaction was performed for 0.5-3h (a shaking speed being 1000rpm) at 16°C on a metal bath oscillator. The microspheres were collected to a 50mL centrifuge tube and were washed for three times with 10mM Tris-HCl(pH8), supernatant was carefully removed after centrifugation, and then the silicon dioxide microspheres were resuspended with enzyme-free water.
5. **Barcode 3 connection reaction:** The resuspended silicon dioxide microspheres from step 4 were uniformly dispensed in the 96-well plate. Then, 100µM of T4 connection buffer, 5µL of 500U/µL 500U/µL T4 ligase, and 5µL of the Barcode 3 primer (primer 4) were added, bringing the total volume to 50µL with water. The reaction was performed at 16°C for 0.5-3 hours on the metal bath oscillator set to a shaking speed of 300-2000rpm. After the reaction ended, the silicon dioxide microspheres were collected to a 50mL centrifuge tube and washed once with 10mM Tris-HCL (pH8). Then, 0.1-2M NaOH was added to denature the primer 3 (a complementary strand of Barcode 2) by incubating1-5 min for each time. After 1-5 cycles, the primer 3 was completely removed in the final cycle; Then microspheres were washed to resuspend for 1-3 times with enzyme-free water, and 10mL of TE-TW(0.01%Tween-20), which were dispensed in the centrifuge tubes and preserved at 4°C.

In this embodiment, various silicon dioxide microspheres with Barcodes can be obtained.

In this embodiment, 1728(12×12×12) or even 452984831(768×768×768) Barcode silicon dioxide microspheres were synthesized, decoding was performed subsequently for preparation of a spatial transcriptome chip.

Part of primer sequences designed and synthesized in this embodiment are shown in Table 4.

**Table 4: Synthesized primers of silicon dioxide microsphere sequences coded with long sequences synthesized by ligation method.**

| Sequence name | Base sequence (5'-3') |
|---|---|
| Primer 1-1 | ctacacgacgctcttccgatctctgcgctcacaatactag (SEQ ID NO: 17) |
| Primer 1-2 | ctacacgacgctcttccgatctcgtaagggcatggttgagt (SEQ ID NO: 18) |
| Primer 1-3 | ctacacgacgctcttccgatctctgtcaagaacgttgacgtcatcgcag (SEQ ID NO: 19) |
| Primer 1-4 | ctacacgacgctcttccgatctcgtcagcttactttgctgtcatcgcagagtactacgt (SEQ ID NO: 4) |
| Primer 2-1 | cgggattagctcaacgagt (SEQ ID NO: 20) |
| Primer 3-1-RC1 | gactcgttgagctaatcccgacgtagtactctgcgatgac (SEQ ID NO: 21) |
| Primer 3-1-RC2 | cgactcgttgagctaatcccgacgtagtactctgcgatgac (SEQ ID NO: 22) |
| Primer 3-1-RC3 | tagtgagtcgactcgttgagctaatcccgacgtagtactctgcgatgac (SEQ ID NO: 23) |
| Primer 3-1-RC4 | caactcactgtagtgagtcgactcgttgagctaatcccgacgtagtactctgcgatgac (SEQ ID NO: 9) |
| Primer 4 | cgactcactacagtgagttggctcgacatgtatcctcatnnnnnnnnnnnntttttttttttttttttttttttttttttvn (SEQ ID NO: 24) |

| | |
|---|---|
| Note: the primers in Table 4 involved different lengths of Linker 1 and Linker 2, but were not limited to combinations of the lengths shown above, with a linker length of 1-20 nt. | |

### Embodiment 6 Effect of evaluating microsphere synthesis by connection method using fluorescence hybridization method.

A DNA probe hybridization solution was prepared, and then hybridized with the synthesized microspheres. Incubation was performed for 15 min at 50°C. After hybridization was finished, the microspheres were washed with a wash buffer, and uniformly spread on a glass slide. The microspheres were aired and then scanned using a scanner. Image analysis was processed under the same parameters after scanning. It can be seen that, under the same parameters, strong fluorescence signals were detected from the microspheres synthesized by the connection method, and microspheres synthesized by a CN114410764A method exhibited lower fluorescence intensity.

A quality testing probe sequence used was 5'-cy5-AAAAAAAAAAAAAAA-3' (SEQ ID NO: 25).

In the present invention, a fluorescence quality testing diagram of the silicon dioxide microspheres coded with long sequences synthesized by the connection method was shown in Fig. 6.

Note: the effects of combining Linker 1 and Linker 2 of various lengths shown in Table 4 were slightly different. The combination with the weakest effect was primer liner 1 with a length of 1 nt and linker 2 with a length of 1 nt. All other combinations were better than the 1&1 combination. A fluorescence quality testing diagram of the 1&1 combination is shown in Fig. 6.

A fluorescence quality testing diagram of silicon dioxide microspheres coded with long sequences synthesized in CN114410764A was shown in Fig. 7.

### Embodiment 7 A method for synthesizing silicon dioxide microspheres coded with long sequences.

The method for synthesizing silicon dioxide microspheres coded with long sequences provided in this embodiment includes the following steps.
1. Activation of carboxylated silicon dioxide
   1.09 mg of EDC and 0.65 mg of NHS were accurately weighed. At the same time 0.1M MES was prepared, and 100 µL of the MES was used to dissolve the well-weighed EDC and NHS, forming an EDC and NHS mixed solution. Meanwhile, 100 µL of carboxylated silicon dioxide microspheres (50 mg/mL) were taken and washed twice with a prepared MES solution. Then, the EDC and NHS mixed solution was added to the microspheres to make a final reaction volume of 100 µL. The microspheres were placed at room temperature and oscillated in a metal bath(2000rpm) for 30 minutes.
2. Connection of barcode 1 amino-modified oligonucleotide strand (primer 1)
   After the reaction, the microspheres were uniformly dispensed into 5 tubes, then 2.5µL of amino-modified oligonucleotide (dissolved in 0.1M MES, with a final concentration of 50µM) was added to each tube. The contents were mixed through blowing, and then the metal bath was oscillated (2000rpm) for reaction overnight at room temperature. After the reaction ended, the microspheres were collected to 1 mLof a 0.1M PBS containing 0.02% tween 20, and centrifuged . The supernatant was carefully removed, and then the microspheres were washed twice in 1ml of a TE buffer (pH 8.0).
3. barcode 2 connection (primer 2)
   The microspheres were washed with water and uniformly dispensed into 5 tubes, then 1µL of BC2 (dissolved in the TE buffer with pH 8.0, with a final concentration being 50µM) and 5µL of a 10×Klenow enzyme reaction buffer were added, and a final total volume was 45µL. A oligonucleotide sequence in each tube included a reverse complementary sequence of linker 2, a reverse complementary sequence of unique barcode, and a reverse complementary sequence of linker 1. The metal bath was oscillated (2000rpm) to operate the following procedures.
   85°C 5min
   80°C 3min
   75°C 3min
   70°C 3min
   65°C 3min
   60°C 3min
   55°C 3min
   50°C 3min
   45 °C 3min
   40 °C 3min
   35 °C 3min
   50°C 10min
   4µL of 2.5mM dNTPs and 1µL of 5U/µL Klenow enzyme were added to each tube after the procedures were completed, and reaction was performed at 37°C at 2000rpm for 1h.
   After the reaction ended, the microspheres were collected to the 0.1M PBS containing 0.02% tween 20, centrifugation was performed, supernatant was carefully removed, then the microspheres were washed twice in the TE buffer (pH 8.0), then 0.1M NaOH was added and stood for 2 min, the reverse complementary sequence of linker 1 was removed, supernatant was removed rapidly after centrifugation, and the operation was repeated twice.
4. barcode 3 (primer 3) and UMI connection
   A ligation step of barcode 3 was the same as that of barcode 2. The added oligonucleotide sequences included a reverse complementary sequence of linker 3, a reverse complementary sequence of a unique barcode, and a reverse complementary sequence of linker 2. After a barcode 3 ligation was cleaned, 2µL of UMI was then added for the same processing, and the added UMI sequences included a poly A tail, the UMI sequence, and the reverse complementary sequence of Linker 3. The barcode 2 ligation step was repeated. The microspheres were stored in a TE-TW solution (10mM Tris pH 8.0; 1mM EDTA, 0.01 % tween 20), and storage was performed at 4°C.

Experimental results were shown in Fig. 8, and various silicon dioxide microspheres coded with long sequences might be obtained.

There was a total of 384×384×384 silicon dioxide microspheres (the number of finally-synthesized microspheres was more than 50 million), which might be used for subsequent preparation of a biological chip.

Sequences involved in this embodiment were shown in Table 5 (SEQ ID NOs: 1-16).

**Table 5:**

| Sequence name | Base sequence (5'-3') |
|---|---|
| Primer1-1 | ctacacgacgctcttccgatctctgcgctcacaatactagtcatcgagagtactacgt (SEQ ID NO: 1) |
| Primer1-2 | ctacacgacgctcttccgatctcgtaagggcatggttgagtcatcgcagagtactacgt (SEQ ID NO: 2) |
| Primer1-3 | ctacacgacgctcttccgatctctgtcaagaacgttgacgtcatcgcagagtactacgt (SEQ ID NO: 3) |
| Primer1-4 | ctacacgacgctcttccgatctcgatcagcttactttgctgtcatcgcagagtactacgt (SEQ ID NO: 4) |
| Primer1-5 | ctacacgacgctcttccgatctacattgcaaggtgcacggtcatcgcagagtactacgt (SEQ ID NO: 5) |
| Primer2-1 | caactcactgtagtgagtcggattgagagctcctgttcgacgtagtactctgcgatgac (SEQ ID NO: 6) |
| Primer2-2 | caactcactgtagtgagtcgttgtgcggaacgtgaccatacgtagtactctgcgatgac (SEQ ID NO: 7) |
| Primer2-3 | caactcactgtagtgagtcgggaaagattctatccgtcgacgtagtactctgcgatgac (SEQ ID NO: 8) |
| Primer2-4 | caactcactgtagtgagtcgactcgttgagctaatcccgacgtagtactctgcgatgac (SEQ ID NO: 9) |
| Primer2-5 | caactcactgtagtgagtcgcccgggtcctaactattgcacgtagtactctgcgatgac (SEQ ID NO: 10) |
| Primer3-1 | ccagacgatcgtgtcactgacaatacgcatctcacagaccaactcactgtagtgagtcg (SEQ ID NO: 11) |
| Primer3-2 | ccagacgatcgtgtcactgatgtatacgtccgctgtcaacaactcactgtagtgagtcg (SEQ ID NO: 12) |
| Primer3-3 | ccagacgatcgtgtcactgaatgaggatacatgtcgagccaactcactgtagtgagtcg (SEQ ID NO: 13) |
| Primer3-4 | ccagacgatcgtgtcactgagcctacttaaccgccagatcaactcactgtagtgagtcg (SEQ ID NO: 14) |
| Primer3-5 | ccagacgatcgtgtcactgaaagccagctcagctacttccaactcactgtagtgagtcg (SEQ ID NO: 15) |
| UMI Primer | nbaaaaaaaaaaaaaaaaaaaaaaaaaaaaaannnnnnnnnnnnnnnnnnnnccagacgatcgtgtcactga (SEQ ID NO: 16) |

| | |
|---|---|
| Note: N and B are degenerate bases, N represents A, T, C, or G, and B represents G, T, or C. | |

### Embodiment 8 Three-color fluorescence decoding technology based on subcellular-level biological chip.

### 1. Chip preparation

As shown in Fig. 25, three groups of primer sequences were designed and synthesized, which respectively were a primer 1 (barcode1), a primer 2 (barcode2), and a primer 3 (barcode3), and each group of primers included 384 sequences. After the primer sequences were synthesized, the primer 1 sequence was first connected to carboxylated silicon dioxide microspheres through an amino carboxyl condensation reaction, the microspheres were mixed and cleaned after connection was completed, and were uniformly divided into 384 portions, a primer 2 sequence was added to each portion for a ligation reaction, and the operation was repeated until a primer 3 sequence was ligated. The prepared microspheres were uniformly spread on a microporous glass plate after being mixed, and were immobilized at well positions, so as to obtain a spatial chip.

### 2. Decoding probe design

The prepared chip needed to be decoded for a plurality of rounds such that a sequence structure carried by the microsphere in each well position was finally determined. Decoding probes were single-stranded oligonucleotide structures respectively complementary to the primer 1, the primer 2, and the primer 3 carried by the microspheres. The decoding probes marked with fluorescence dyes selected three fluorescence dyes from fluorescence dyes such as DAPI, FITC, Alexa fluor 488, CY2, Cy3, Cy5, CY5.5, TRITC, Cy7, etc. Furthermore, probes not marked with fluorescence dyes were determined to be dark. The three fluorescence dyes marked Barcode 1, Barcode 2, and Barcode 3, respectively.

### 3. Chip hybridization

A decoding hybridization solution was first prepared for chip hybridization. There was total 50-200µL of the hybridization solution per well per round, including 40-190µL of a hybridization buffer (1-10mM NaCl, 2-5mM Tris-HCl, 1-3mM MgCl₂, and 0.5-5mM DTT), and 10µL(1nM-50nM) of mixed probes with three colors. After the hybridization solution was prepared, the spread chip was placed in a card clamp, the hybridization solution was added in well positions to be decoded, and the hybridization solution was ensured to cover surfaces of entire well positions. Then the chip was placed in a 37-60°C metal bath, and reaction was performed for 5-20 min. The hybridization solution was removed after reaction ended, excess probes were cleaned with NFW, the chip was dried, and then fluorescence signals in the well positions were collected by a fluorescence scanner.

### 3. DNA denaturation and cleaning

After the chip was subjected to fluorescence collection, 100µL of 0.1-2M NaOH was taken and put on the well positions and was allowed to stand for 1-10 min to ensure complete DNA denaturation, the operation was repeated for 2-3 times, then cleaning was performed for 1-3 times with NFW, and a hybridization probe was ensured to be cleaned to prevent a next round of reaction from being affected. The chip was cleaned and dried, then a next round of decoding probes was hybridized again, and the operation was repeated until 2-9 rounds of decoding flows were completed.

### 4. Chip decoding

After the chip was subjected to 2-9 rounds of decoding, the fluorescence signal of each well position of each round of fluorescent images collected was extracted, and a sequence structure corresponding to each well position was obtained according to the arrangement of the colors of each round of fluorescent tags in each well position, so as to achieve chip decoding.

**Table 6: Fluorescently-labeled probes corresponding to each round of decoding cycles**

| Decoding cycle | Barcode | Black | Fluorescence 1 | Fluorescence 2 | Fluorescence 3 |
|---|---|---|---|---|---|
| 1 | Bar1-1 | | √ | | |
| | Bar1-2 | | √ | | |
| | Bar1-3 | √ | | | |
| | Bar1-4 | √ | | | |
| | Bar2-1 | | | √ | |
| | Bar2-2 | | | √ | |
| | Bar2-3 | √ | | | |
| | Bar2-4 | √ | | | |
| | Bar3-1 | | | | √ |
| | Bar3-2 | | | | √ |
| | Bar3-3 | √ | | | |
| | Bar3-4 | √ | | | |
| 2 | Bar1-1 | | √ | | |
| | Bar1-2 | √ | | | |
| | Bar1-3 | | √ | | |
| | Bar1-4 | √ | | | |
| | Bar2-1 | | | √ | |
| | Bar2-2 | √ | | | |
| | Bar2-3 | | | √ | |
| | Bar2-4 | √ | | | |
| | Bar3-1 | | | | √ |
| | Bar3-2 | √ | | | |
| | Bar3-3 | | | | √ |
| | Bar3-4 | √ | | | |

Table 7 Fluorescence signal combination corresponding to each primer sequence.

**Table 7**

| Barcode | Decoding mode |
|---|---|
| Bar1-1 | Fluorescence 1-Fluorescence 1 |
| Bar1-2 | Fluorescence 1-Black |
| Bar1-3 | Black-Fluorescence 1 |
| Bar1-4 | Black-Black |
| Bar2-1 | Fluorescence 2-Fluorescence 2 |
| Bar2-2 | Fluorescence 2-Black |
| Bar2-3 | Black-Fluorescence 2 |
| Bar2-4 | Black-Black |
| Bar3-1 | Fluorescence 3-Fluorescence 3 |
| Bar3-2 | Fluorescence 3-Black |
| Bar3-3 | Black-Fluorescence 3 |
| Bar3-4 | Black-Black |

For example, the primer 1, the primer 2, and the primer 3 respectively included 4 primer sequences. Table 8 showed sequences of the primers 1, 2, and 3, and sequences (reverse complementary sequences of barcode) of the decoding probes. The decoding probes needed to be marked and then mixed for use.

**Table 8**

| Item | Sequence (5'-3') |
|---|---|
| Primer1-1 | ctacacgacgctcttccgatcttttagctctcctatggctacgt (SEQ ID NO: 26) |
| Primer1-2 | ctacacgacgctcttccgatctcgagctcaaggatatcgtacgt (SEQ ID NO: 27) |
| Primer1-3 | ctacacgacgctcttccgatctggcgtaaggaccttacgtacgt (SEQ ID NO: 28) |
| Primer1-4 | ctacacgacgctcttccgatcttcgcttacaaatctggttacgt (SEQ ID NO: 29) |
| Primer2-1 | ttcttcacagccagcctag (SEQ ID NO: 30) |
| Primer2-2 | tataggtgacctgcagtaa (SEQ ID NO: 31) |
| Primer2-3 | ccatatacgtgctaccaaa (SEQ ID NO: 32) |
| Primer2-4 | cacatacttgcgggttgag (SEQ ID NO: 33) |
| Primer3-1 | cgactcttgacgtcgaagtccttcgnnnnnnnnnnnnttttttttttttttttttttvn (SEQ ID NO: 34) |
| Primer3-2 | cgactcgaggtacatgctacccgacnnnnnnnnnnnnttttttttttttttttttttvn (SEQ ID NO: 35) |
| Primer3-3 | cgactcatgcatgtgtcagtgtggcnnnnnnnnnnnnttttttttttttttttttttvn (SEQ ID NO: 36) |
| Primer3-4 | cgactccattatgaggtaaccctagnnnnnnnnnnnnttttttttttttttttttttvn (SEQ ID NO: 37) |
| Decoding probeI-1 | gccataggagagctaaa (SEQ ID NO: 38) |
| Decoding probeI-2 | cgatatccttgagctcg (SEQ ID NO: 39) |
| Decoding probeI-3 | cgtaaggtccttacgcc (SEQ ID NO: 40) |
| Decoding probeI-4 | accagatttgtaagcga (SEQ ID NO: 41) |
| Decoding probeII-1 | ctaggctggctgtgaagaa (SEQ ID NO: 42) |
| Decoding probeII-2 | ttactgcaggtcacctata (SEQ ID NO: 43) |
| Decoding probeII-3 | tttggtagcacgtatatgg (SEQ ID NO: 44) |
| Decoding probeII-4 | ctcaacccgcaagtatgtg (SEQ ID NO: 45) |
| Decoding probeIII-1 | cgaaggacttcgacgtcaa (SEQ ID NO: 46) |
| Decoding probeIII-2 | gtcgggtagcatgtacctc (SEQ ID NO: 47) |
| Decoding probeIII-3 | gccacactgacacatgcat (SEQ ID NO: 48) |
| Decoding probeIII-4 | ctagggttacctcataatg (SEQ ID NO: 49) |

V represents A, G, or C, and N represents A, T, G, or C.

The above are only the preferred embodiments of the present invention and are not intended to limit the present invention. For those skilled in the art, the present invention may have various modifications and variations. Any modifications, equivalent replacements, improvements and the like made within the spirit and principle of the present invention all fall within the scope of protection of the present invention.

## Claims

1. A method for preparing a microsphere chip, comprising the following steps:
1) cleaning a silicon dioxide glass slide with micropores;
2) uniformly spin-coating an ultraviolet curing adhesive on a surface of the cleaned glass slide, then performing ultraviolet radiation, and forming a uniform thin film on the surface of the glass slide;
3) preparing a silicon dioxide microsphere solution with a concentration of 2×10⁵-3×10⁵/µL, then dropwise adding the microsphere solution in the middle of the glass slide with the thin film, and opening a spin-coating apparatus for centrifugation, so as to make microspheres drop into micropores of the glass slide;
4) removing liquid left on the surface of the glass slide, washing with ultrapure water, and then drying; and
5) cleaning the surface of the glass slide with a brush, wherein
the diameters of the micropores are similar to the diameters of the silicon dioxide microspheres.

2. The method according to claim 1, wherein the ultraviolet curing adhesive is a Feifanli 3217 UV glue produced by Mizhan Technology Co., Ltd.; the thickness of the thin film is 1 nm-3 µm, and preferably 1-3 µm.

3. The method according to claim 1, wherein the diameters of the silicon dioxide microspheres are 2-3 µm.

4. The method according to claim 3, wherein the depths of the micropores on the silicon dioxide glass slide are 1.5-2.5 µm, the micropores uniformly distributed on the glass slide, and a distance between centers of the two adjacent micropores is 4-6 µm, calculated according to a total area 7 mm×7 mm of the glass slide.

5. The method according to claim 1, wherein the centrifugation in step 3) is performed with a plate centrifuge at a rotary speed of 1000 rpm-3000 rpm for10 seconds to 1 minute.

6. The method according to claim 1, wherein a reagent for preparing the silicon dioxide microsphere solution in step 3) is the ultrapure water, a DMSO solution with a concentration of 5-20%, or an ultraviolet curing adhesive solution with a concentration of 2.5-10‰.

7. The method according to claim 1, wherein step 1) comprises: soaking the silicon dioxide glass slide in a Piranha Solution for 30 minutes, then washing successively with the ultrapure water and anhydrous ethanol, and subsequently air drying, wherein
the Piranha Solution is a mixture of a concentrated sulfuric acid and a hydrogen peroxide solution in a volume ratio of 7:3.

8. The method according to any one of claims 1 to 7, wherein the silicon dioxide microspheres are covalently bonded with nucleic acids, proteins, or peptides.

9. The method according to claim 8, wherein the nucleic acids are long coding sequences, and the silicon dioxide microspheres are silicon dioxide microspheres with long coding sequences; and
preferably, a method for preparing the silicon dioxide microspheres with long coding sequences comprises the following steps:
(1) designing four groups of primers, which respectively are a primer 1, a primer 2, a primer 3, and a primer 4, and the primers are all single-stranded oligonucleotides, with the ability to anneal between the primer 2 and the primer 3;
wherein, the primer 1 has an amino group modification at 5' end, and comprises a READ1 sequence, a barcode 1 sequence, and a linker 1 sequence of an Illumina sequencing platform from 5' to 3'; the barcode 1 sequence is a coding sequence with a length of 10-20 nt, and the linker 1 sequence is an auxiliary connection sequence with a length of 1-20 nt;
the primer 2 is a coding sequence with a length of 10-20 nt, i.e. a barcode 2 sequence;
a length of the primer 3 is 12-60 nt, and the primer 3 comprises, a reverse complementary sequence of a linker 2 sequence, a reverse complementary sequence of the barcode 2 sequence, and a reverse complementary sequence of the linker 1 sequence from 5' to 3';
the primer 4 comprises, the linker 2 sequence, a barcode 3 sequence, a UMI sequence (random sequence), and a Poly T sequence from 5' to 3'; and the barcode 3 sequence is a coding sequence with a length of 10-20 nt, the UMI sequence is a random primer with a length of 8-16 nt, and the Poly T sequence has a length of 10-35 nt and comprises a VN sequence at the end, wherein V and N are degenerate bases, V represents A, G or C, and N represents A, T, G, or C;
(2) activation of carboxylated silicon dioxide microspheres: placing the carboxylated silicon dioxide microspheres in an EDC and NHS mixed solution for activation;
(3) mixing the activated microspheres and the primer 1, and performing a condensation reaction to obtain microspheres with different sequences;
(4) performing an annealing reaction between the primer 2 and the primer 3;
(5) mixing an annealing product obtained in step (4) and the microspheres obtained in step (3), and performing a DNA strand ligation reaction; and
(6) mixing the microspheres obtained in step (5) and the primer 4, performing a ligation reaction, thereby obtaining the silicon dioxide microspheres with long coding sequences.

10. The method according to claim 9, wherein step (2) comprises: centrifugating 4-10 mL of the carboxylated silicon dioxide microspheres with a concentration of 0.1-0.5 mg/mL, then precipitating and mixing with 4-10 mL of the EDC and NHS mixed solution, and oscillating overnight at room temperature and 300-2000rpm, wherein,
a method for preparing the EDC and NHS mixed solution comprises: dissolving 10-30 mg of EDC and 5-30 mg of NHS in 1000-10000 µL of 0.1-1M MES, so as to obtain the EDC and NHS mixed solution.

11. The method according to claim 9, wherein step (3) comprises: adding the activated microspheres to a 96-well plate, with 10-40 µL per well, additionally adding 2-10 µL of a primer 1 solution to each well, and oscillating overnight at 20-30°C and 300-2000 rpm; after the reaction ends, washing the microspheres with PBS containing 0.001-0.03% v/v tween-20, then washing the microspheres with a TE buffer, and resuspending the cleaned microspheres with enzyme-free water, wherein
the primer 1 solution is prepared by dissolving the primer 1 in 0.1-1M MES buffer, resulting in a final concentration of 10-100 µM for the Primer 1 solution.

12. The method according to claim 9, wherein step (4) comprises: mixing the primer 2 and the primer 3 in an equal molar ratio, adding a 5× annealing buffer for an annealing reaction; a condition for the annealing reaction comprises: 95°C~ 15°C, annealing at 1-10°C every 1-3 minutes, so as to obtain the annealing product; and
the 5× annealing buffer comprises: 10-50 µL of a 1M Tris-HCl solution, 5-20 µL of 0.5M EDTA, and 50-150 µL of 2M NaCl, making up to 1000 µL with double distilled water.

13. The method according to claim 9, wherein step (5) comprises: adding the microspheres obtained in step (3) to a 96-well plate, with 10-40 µL per well, and additionally adding, to each well, 5 µL of a 5×T4 ligation buffer, 2-10 µL of 100-1000U/µL T4 ligase, and 2-10 µL of 20-100µM the annealing product, making up to 50 µLwith double distilled water; oscillating at 16°C and300-2000rpm for 0.5-3 hours; and then washing the microspheres with a 5-20mM pH8 Tris-HCl solution, and resuspending the cleaned microspheres with enzyme-free water.

14. The method according to claim 9, wherein step (6) comprises: adding the microspheres obtained in step (5) to a 96-well plate, with 10-40 µL per well, and additionally adding, to each well, 5 µL of a 5×T4 ligation buffer, 2-10 µL of 100-1000U/µL T4 ligase, and 2-10 µL of a primer 4 solution, making up to 50 µL with double distilled water; oscillating at 16°C and 300-2000rpm for 0.5-3 hours; and then washing the microspheres with a 10mM pH8 Tris-HCl solution, placing the cleaned microspheres in a 0.1-2M NaOH solution for DNA denaturation, then washing the microspheres with enzyme-free water, finally resuspending the cleaned microspheres with a TE-TW solution, and performing preservation at 4°C, wherein,
the primer 4 solution is prepared by dissolving the primer 4 in a TE buffer with pH 8.0, resulting in a final concentration of 10-100 µM for the Primer 4 solution; and
the TE-TW solution is a TE buffer containing 0.01% Tween-20.

15. The method according to claim 8, wherein the nucleic acids are long DNA sequences, and the silicon dioxide microspheres are silicon dioxide microspheres with long DNA sequences; and
preferably, a method for preparing the silicon dioxide microspheres with long DNA sequences comprises the following steps:
(1) designing four groups of primers, which respectively are a primer 1, a primer 2, a primer 3 and a UMI primer, wherein the primers are all single-stranded oligonucleotides, the lengths of the primer 1, the primer 2, and the primer 3 are the same, with a GC content between 45% and 55%, ensuring that Tm values of each primer are similar; the primer 1 has an amino group modification at 5' end, and comprises a READ1 sequence of an Illumina sequencing platform, a barcode 1 sequence and a linker 1 sequence from 5' to 3'; the primer 2 comprises a reverse complementary sequence of a linker 2 sequence, a reverse complementary sequence of a barcode 2 sequence, and a reverse complementary sequence of the linker 1 sequence from 5' to 3'; the primer 3 comprises a reverse complementary sequence of a linker 3 sequence, a reverse complementary sequence of a barcode 3 sequence, and a reverse complementary sequence of the linker 2 sequence from 5' to 3', the UMI primer comprises a polyA sequence, a UMI sequence (random sequence), and a reverse complementary sequence of the linker 3 sequence from 5' to 3'; and wherein the barcode 1 sequence, the barcode 2 sequence, and the barcode 3 sequence are different barcode sequences, and a length of the polyA sequence is 20-35 nt;
(2) activation of carboxylated silicon dioxide microspheres: placing the carboxylated silicon dioxide microspheres in an EDC and NHS mixed solution for activation;
(3) ligation reaction: putting the activated microspheres in a primer 1 solution, and performing a condensation reaction to obtain silicon dioxide microspheres with different sequences;
(4) synthesis of the long DNA sequences: mixing the microspheres obtained in step (3), the primer 2, and a polymerization reaction reagent, performing a DNA strand extension reaction, and then removing the reverse complementary sequence of the linker 1 sequence; mixing the obtained microspheres, the primer 3, and the polymerization reaction reagent, further performing the DNA strand extension reaction, and then removing the reverse complementary sequence of the linker 2 sequence; and mixing the microspheres, the UMI sequence, and the polymerization reaction reagent, further performing the DNA strand extension reaction, and then removing the reverse complementary sequence of the linker 3 sequence, so as to obtain the silicon dioxide microspheres with long DNA sequences.

16. The method according to claim 15, wherein step (2) comprises: centrifuging 50 mg/mL of the carboxylated silicon dioxide microspheres, then precipitating and mixing the carboxylated silicon dioxide microspheres and 20-100 µL of an EDC and NHS mixed solution, and oscillating at room temperature and 1500-2000rpm for 30 min-1 h, wherein
a method for preparing the EDC and NHS mixed solution comprises: dissolving 1.09 mg of EDC and 0.65 mg of NHS in 100 ul of 0.1M MES, so as to obtain the EDC and NHS mixed solution.

17. The method according to claim 15, wherein step (3) comprises: uniformly mixing the activated microspheres and 2.5 µL of an oligonucleotide chain solution modified by 50µM amino, and oscillating overnight at room temperature at 2000rpm; and centrifugating after the reaction is complete, collecting the microspheres, and washing the microspheres for subsequent synthesis reaction.

18. The method according to claim 17, wherein washing the microspheres comprises: placing the microspheres in 0.1M PBS containing 0.02% tween 20, centrifugating and collecting the microspheres, and then washing the microspheres with a TE buffer with pH 8.0 for twice.

19. The method according to claim 17, wherein the polymerization reaction reagent in step (4) comprises: dNTPs, Klenow enzyme, and a Klenow enzyme reaction buffer.

20. The method according to claim 19, wherein a reaction system for performing DNA strand extension comprises 1 µL of a 50µM primer 2, 5 µL of the 10×Klenow enzyme reaction buffer, 4 µL of the 2.5mM dNTPs, and 1 µL of the 5U/µL Klenow enzyme; and
a reaction condition comprises: performing oscillating for reaction at 37°C at 2000rpm for 0.5-1 hour.

21. The method according to claim 20, wherein a reaction system for the DNA-strand extension of the UMI primer and the microspheres in step (4) comprises 50µM the UMI primer, 5 µ Lof 10×Klenow enzyme reaction buffer, 4 µL of 2.5mM dNTPs, and 1 µL of 5U/µL Klenow enzyme; and
a reaction condition comprises: performing oscillating for reaction at 37°C at 2000rpm for 0.5-1 hour.

22. The method according to any one of claims 15 to 21, wherein a nucleotide sequence of the primer 1 is shown in SEQ ID NOs: 1-5, a nucleotide sequence of the primer 2 is shown in SEQ ID NOs: 6-10, a nucleotide sequence of the primer 3 is shown in SEQ ID NOs: 11-15, and a nucleotide sequence of the UMI primer is shown in SEQ ID NO: 16.

23. The method according to any one of claims 1 to 22, wherein in step 1), the silicon dioxide glass slide with micropores serves as a spatial transcriptome biological chip, and comprises:
a substrate, wherein a plurality of rectangular microporous regions are formed on the substrate, a plurality of sub-rectangular microporous regions are formed within each rectangular microporous region, a plurality of microporous structures are uniformly distributed in each sub-rectangular microporous region, the microporous structure is configured to place coded microspheres, and the substrate is a glass substrate.

24. The method according to claim 23, wherein a gap is formed between the adjacent sub-rectangular microporous regions, and the size of each gap is the same.

25. The method according to claim 24, wherein the width of each gap ranges from 2 µm to 20 µm.

26. The method according to claim 23, wherein at least one of the sub-rectangular microporous regions located at the four right angels of the rectangular microporous region is provided with a marker.

27. The method according to claim 26, wherein notches are formed in the sub-rectangular microporous regions located at any three of the four right angles of the rectangular microporous region, so as to form the marker.

28. The method according to claim 27, wherein the notch is located at any one of the right regions of the sub-rectangular microporous region.

29. The method according to any one of claims 23 to 28, wherein the diameter of the microporous structure ranges from 1 µm to 10 µm.

30. The method according to any one of claims 23 to 28, wherein the length and width of the rectangular microporous region both range from 5 mm to 20 mm.

31. The method according to any one of claims 23 to 28, wherein the length and width of the sub-rectangular microporous region both range from 100 µm to 300 µm.

32. The method according to any one of claims 23 to 28, wherein the glass substrate is etched to form the microporous structure.

33. The method according to any one of claims 1 to 22, wherein in step 1), the silicon dioxide glass slide with micropores is a spatial transcriptome biological chip, and comprises:
a transparent substrate, wherein the transparent substrate is subjected to photolithography and etching to form a microporous region, a microporous structure is formed within the microporous region, so as to place coded microspheres with primers; at least one sub-region is formed in the microporous region, the sub-region is a circular region or a polygonal region, and the transparent substrate is a glass substrate.

34. The method according to claim 33, wherein a plurality of micro-units are formed within the sub-region, the adjacent micro-units are arranged at intervals, and the micro-units are repeatedly arranged in circles or polygons.

35. The method according to claim 34, wherein a notch is formed at an edge and/or a corner of the micro-unit, so as to form an azimuthal marker.

36. The method according to claim 34, wherein a distance between the adjacent micro-units is between 0 µm and 40 µm.

37. The method according to any one of claims 33 to 36, wherein a diameter of the microporous structure ranges from 0.1 µm to 10 µm, and a distance between central points of adjacent two micropores is between 0.1 µm and 20 µm.

38. The method according to any one of claims 33 to 36, wherein a long side of the transparent substrate ranges from 10 to 100 mm, and a short side ranges from 5 to 50 mm.

39. The method according to any one of claims 33 to 36, wherein a size of the sub-region ranges from 9mm² to 1875mm².

40. The method according to any one of claims 33 to 36, wherein the microporous structure comprises an expansion portion located on a surface of the transparent substrate and a shrinkage portion located inside the transparent substrate, the expansion portion and the shrinkage portion are interconnected along a depth direction of the transparent substrate, so as to form the microporous structure on the transparent substrate.

41. The method according to any one of claims 33 to 36, wherein the transparent substrate comprises a first surface and a second surface, and the first surface and the second surface are respectively subjected to photolithography and etching to form the microporous regions; and
the first surface and the second surface are two opposite surfaces of the transparent substrate.

42. A three-color fluorescence decoding method based on a subcellular-level biological chip, comprising the following steps:
A, preparation of a biological chip: immobilizing silicon dioxide microspheres, connected with a primer 1, a primer 2, and a primer 3, at well positions of a well plate using the method according to any one of claims 9 to 22, so as to obtain the biological chip;
B, hybridization of the chip and a decoding probe: mixing a decoding probe I, a decoding probe II, and a decoding probe III, which have different fluorescent tags, then performing a hybridization reaction with the biological chip, and then detecting corresponding fluorescence signals;
C, DNA denaturation and cleaning: denaturing the hybridized biological chip with sodium hydroxide, washing and drying the melted biological chip, and then re-hybridizing a next round of decoding probes again; and
D, repeating steps B-C for 2-9 times, and achieving chip decoding,
wherein the primer 1, the primer 2, and the primer 3 comprises 4-384 primer sequences, respectively; the primer 1, the primer 2, and the primer 3 are connected in series in sequence according to 5'-3'; and
the decoding probe I is a single-stranded oligonucleotide complementary to the primer 1, the decoding probe II is a single-stranded oligonucleotide complementary to the primer 2, and the decoding probe III is a single-stranded oligonucleotide complementary to the primer 3.

43. The method according to claim 42, wherein the silicon dioxide microsphere in the step A are carboxylated silicon dioxide microspheres.

44. The method according to claim 43, wherein the step A comprises: connecting each of the 4-384 primer sequences of the primer 1 to the carboxylated silicon dioxide microspheres through an amino-carboxyl condensation reaction, mixing and washing the microspheres after connection is completed, and uniformly dividing the microspheres into 4-384 portions; performing a ligation reaction on each portion added with 4-384 primer sequences of the primer 2, mixing and washing the microspheres after connection is completed, and uniformly dividing the microspheres into 4-384 portions; performing the connection reaction on each portion added with 4-384 primer sequences of the primer 3, uniformly mixing and spreading the microspheres on a microporous glass plate after connection is completed, and immobilizing the microporous glass plate at the well positions, so as to obtain the biological chip.

45. The method according to claim 42, wherein the fluorescent tag used in the step B is selected from the group consisting of DAPI, FITC, Alexa fluor 488, Cy2, Cy3, Cy5, Cy5.5, TRITC, and Cy7.

46. The method according to claim 42, wherein the hybridization reaction in the step B is performed in a hybridization buffer; and the hybridization buffer comprise 1-10mM NaCl, 2-5mM Tris-HCl, 1-3mM MgCl₂ and 0.5-5mM DTT.

47. The method according to claim 46, wherein in the step B, a concentration of the mixed decoding probe is 1 nM-50 nM, 10 µL of the mixed decoding probe is mixed with 40-190 µL of the hybridization buffer, and the mixture is added to the well positions, so as to perform the hybridization reaction with the biological chip.

48. The method according to claim 47, wherein a condition of performing the hybridization reaction in the step B comprises: performing the reaction at 37-60°C for 5-20 min.

49. The method according to claim 47, wherein the step C comprises:
C1, adding 100 µL of a 0.1-2M sodium hydroxide solution to each well position, allowing the solution to stand for 1-10 minutes, and then pouring the solution in the well positions;
C2, repeating step C1 for 2-3 times to ensure complete DNA denaturation; and
C3, washing the melted biological chip with NFW for 1-3 times, then drying and hybridizing with a next round of decoding probes.

50. (Cancelled) Use of the method according to any one of claims 45 to 49 in spatial omics research at a subcellular level.
